# FASCICULE DE BREVET EUROPEEN

(11) **EP 0 580 476 B1**
(45) Date de publication et mention de la délivrance du brevet: **16.04.1997**
(21) Numéro de dépôt: 93401810.2
(22) Date de dépôt: 13.07.1993
(51) Int. Cl.: C07C 59/42, C07C 51/09

(54) **Procédé de fabrication de 6E-leucotriène B4 et produits intermédiaires dudit procédé de fabrication**
Verfahren zur Herstellung von 6E-Leucotrien B4 und Zwischenprodukte aus diesem Herstellungsverfahren
Process for producing 6E-leucotriene B4 and intermediates from this production process

(30) Priorité: 21.07.1992 FR 9208978
(43) Date de publication de la demande: 26.01.1994
(73) Titulaire: L'OREAL, 75008 Paris (FR)
(72) Inventeur: MAIGNAN Jean, 93290 TREMBLAY-LES-GONESSES (FR); SOLLADIE Guy, 67000 STRASBOURG (FR); STONE Guy, 67000 STRASBOURG (FR)
(74) Mandataire: Peuscet, Jacques

(56) Documents cités:
- US-A- 4 873 024
- CHEMICAL ABSTRACTS, vol. 103, no. 17, 28 octobre 1985, abstract no. 141711q, page 698, Columbus, Ohio, US; K. GREEN et al, "Natural acetylenes. Part 59. Synthesis of (-)-(E,S)-dodec-4-ene-6,8,10-triyn-3-ol, the enantiomer of a metabolite of the fungus Peniophora resinosa Jackson and Dearden"

## Description

La présente invention concerne un procédé de fabrication du leucotriène B₄, ci-après désigné par LTB₄, sous sa configuration 6-trans (dite aussi 6 E) et des produits intermédiaires obtenus au cours de cette fabrication.

On sait que les leucotriènes sont des métabolites de l'acide arachidonique dans les leucocytes. L'acide arachidonique est transformé en un composé époxyde instable dénommé leucotriène A₄, qui est lui-même transformé par voie enzymatique en leucotriène B₄ (LTB₄). Le LTB₄ provoque l'adhésion et le mouvement chemotactique des leucocytes et stimule l'aggrégation, la libération d'enzymes et la formation de superoxydes dans les neutrophiles. Le LTB₄ est donc impliqué dans les processus inflammatoires.

Le LTB₄ peut difficilement être préparé à partir de ses sources naturelles et il est, de plus en plus, demandé en pharmacie dans le cadre de recherches biologiques sur les processus inflammatoires.

On a donc essayé de préparer le LTB₄ par synthèse, mais le problème est d'obtenir un produit avec une structure stéréochimique identique à celle du produit biologique, c'est-à-dire d'obtenir le LTB₄ sous sa forme 6-trans qui a pour formule:

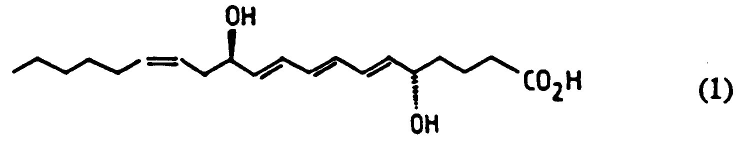

On a proposé un grand nombre de procédés de synthèse des leucotriènes, par exemple dans J. Amer. Chem. Soc. (1980), 102, 7984 ; Tetrahedron letters (1987), 5849 ; J. Org. Chem. (1990), 55, 5324 ; Tetrahedron letters (1983), 24, 409 ; Tetrahedron letters (1986), 5857 ; J. Org. Chem. (1992), 651 trahedron letters (1981), 1077; J. Amer. Chem. Soc. (1984), 106, 3548 ; Tetrahedron letters (1982), 2631; Tetrahedron letters (1982), 23, 739 ; Tetrahedron letters (1986), 27, 4161; J. Org. Chem. (1989), 54, 2409 ; J. Org. Chem. (1988), 53, 265; J. Org. Chem. (1988), 53, 267 et J. Org. Chem. (1986), 51, 1253.

Dans tous les procédés décrits dans les documents ci-dessus, on obtient le LTB₄ sous forme de mélanges d'isomères, le 6 E-LTB₄ étant toujours présent dans de faibles proportions.

L'article de Corey et collaborateurs publié dans Tetrahedron Letters (1981), vol 22, n° 17 pages 1587-1590, décrit un procédé de synthèse du 6 E-LTB₄ par réaction d'un sel de phosphonium de formule: 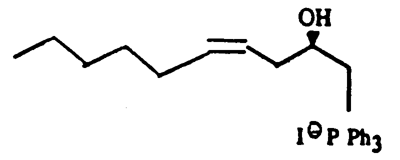 avec le diène de formule: 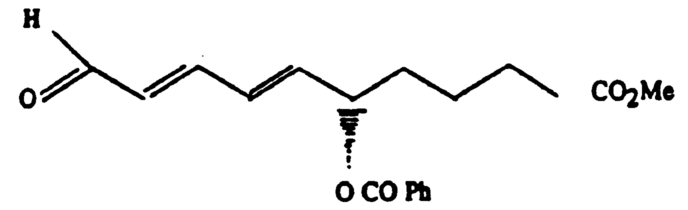 dans le tétrahydrofuranne en présence de n-butyllithium et d'hexaméthyl phosphoretriamide, cette réaction étant suivie d'une saponification (dans ces formules, Me = méthyle et Ph = phényle).

Pour la mise en oeuvre de ce procédé, le sel de phosphonium est obtenu à partir du (R,R) (+) diméthyltartrate par un procédé en 9 étapes et le diène est obtenu à partir du deoxyribose par un procédé en 8 étapes. Le procédé de synthèse de Corey est donc un procédé long et coûteux, qui ne permet pas d'obtenir un rendement satisfaisant. Bien que le produit obtenu ait une pureté stéréochimique en 6 E-LTB₄, qui est meilleure que dans les procédés cités ci-dessus, elle n'est pas encore entièrement satisfaisante.

La présente invention a pour but de décrire un procédé de préparation de LTB₄ qui est plus rapide, donc moins coûteux, et qui a une plus grande stéréospécificité 6-trans et un meilleur rendement.

La présente invention a donc pour objet un procédé de préparation de leucotriène B₄ (LTB₄) sous forme 6-trans (6-E) ayant pour formule : 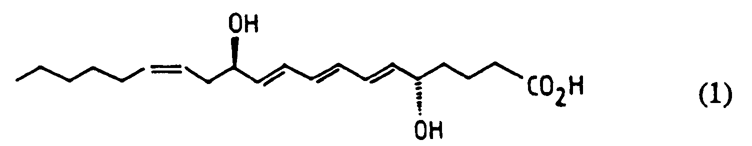 dans lequel on prépare un butadiène diol de formule : 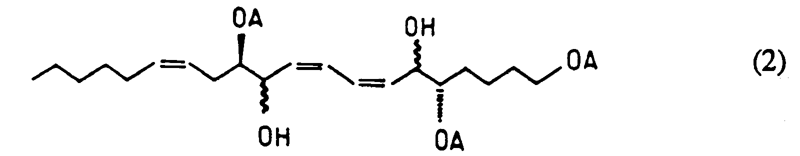 formule dans laquelle A est un groupe protecteur silylé ,de préférence un groupe tert-butyl diméthylsilyle (ci-après indiqué TBDMS) ; on estérifie les groupes hydroxyle pour obtenir un groupe OCOB, B étant un radical phényle ou phényle substitué par un radical alkyle ou alcoxy en C₁-C₆, de préférence un radical phényle non substitué, pour obtenir le diester de formule : 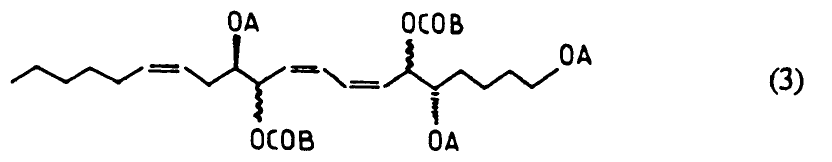 et on soumet le diester de formule (3) à une élimination réductrice à l'aide de titane basse valence [Ti(0)] ou d'un amalgame alcalin pour obtenir le triéther 6-trans de formule : 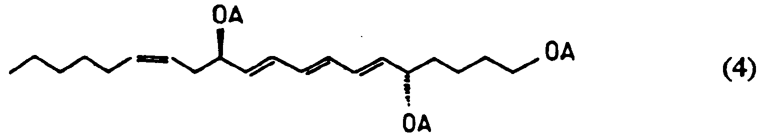 que l'on transforme ensuite par des procédés connus en LTB₄ 6-trans par transformation des groupes -OA en positions 5 et 12 en groupes -OH et du groupe -CH₂OA en position 1 en groupe -COOH.

L'amalgame d'un métal alcalin utilisé est de préférence un amalgame de sodium.
L'utilisation de titane basse valence "Ti(0)" obtenu par mélange de trichlorure de titane et d'hydrure d'aluminium et de lithium (TiCl₃/LiAlH₄) est connue. Ce titane basse valence est symbolisé dans la présente demande par "Ti(0)" bien que la valence du titane ne soit pas toujours exactement 0. Il a été utilisé pour la première fois par Mc Murry [Acc. Chem. Res., Vol 7, n° 9 (1974)] pour le couplage réductif de composés carbonylés en oléfines selon le schéma réactionnel :

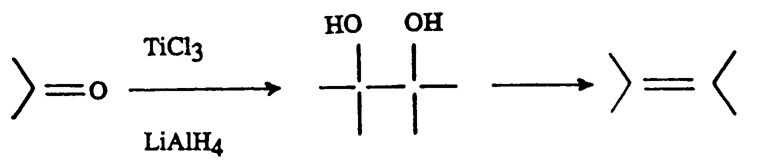

H. M. Walborsky et Wüst H.H. [J. Am. Chem. Soc. (1982), 104, 5807] ont ensuite utilisé Ti(0) pour préparer des diènes 1,3 à partir de diols allyliques.

L'étude de la stéréochimie de cette réaction et son application à la synthèse de la vitamine A et du 13-cis rétinol ont été décrites par G. Solladié et autres dans J. Org. Chem. (1989), 54, 2620. On a constaté que, dans la synthèse de la vitamine A, le procédé d'élimination réductrice était stéréospécifique, c'est-à-dire que lorsqu'on part d'un diol 13-trans, on obtient un composé tout-trans. De même, si on part d'un diol 13-cis, on obtient après réduction un composé 13-cis.

Selon la présente demande, on a maintenant trouvé que l'on pouvait réduire de façon stéréospécifique un diol dérivé d'un diène, par réduction à l'aide de Ti(0) pour obtenir un triène à condition que les groupes hydroxyle du diol ne soient pas libres mais soient bloqués sous forme d'ester. En d'autres termes, selon la présente demande, on fait réagir un diène ayant deux doubles liaisons conjuguées, de formule : 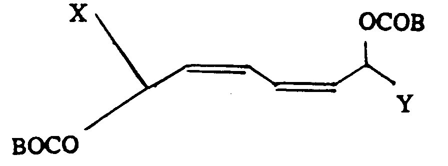 pour obtenir un triène ayant trois doubles liaisons conjuguées :

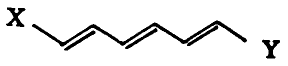

On a constaté que cette réaction de réduction à l'aide de titane basse valence [Ti(0)] était stéréospécifique, c'est-à-dire qu'à partir d'un ester diénique 6-trans de formule (3), on obtenait le triéther triénique de formule (4) sous sa forme 6-trans, 8-trans, 10-trans, avec un rendement voisin de 99 %.

On a également constaté que la réduction par un amalgame alcalin était stéréospécifique.

Selon l'invention, l'élimination réductrice à l'aide de titane basse valence [Ti(0)] est, de préférence, effectuée dans un solvant, en particulier le tétrahydrofuranne, à une température comprise entre 50 et 70°C.

Selon l'invention le triéther triénique de formule (4) est de préférence, transformé en LTB₄ 6-trans par le procédé suivant :
1) réaction du triéther de formule (4) avec une solution de fluorure de tétra-n-butylammonium dans un solvant, en particulier le tétrahydrofuranne, à une température comprise entre -10 et +20°C pour obtenir le triol de formule : 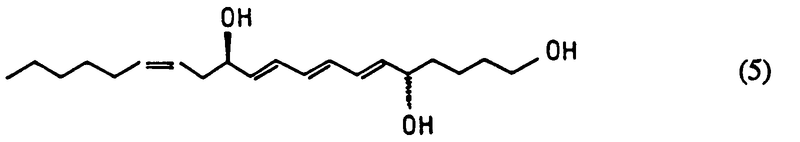
2) réaction du triol de formule (5) avec RuCl₂(PPh₃)₂ (où Ph = phényle), sous argon, à une température comprise entre 10 et 30°C pour obtenir la lactone de formule : 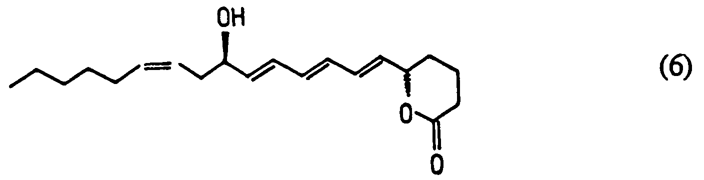 et
3) traitement de la lactone de formule (6) par LiOH en solution dans un solvant, en particulier le tétrahydrofuranne, à une température inférieure à 0°C.

Selon la présente invention, le butadiènediol de formule (2) est, de préférence, préparé par hydrogénation du composé diacétylénique de formule : 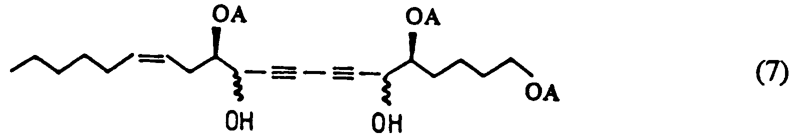 (où A a la même signification que précédemment) sous argon, en présence de Zn activé en solution dans un mélange eau/méthanol, le composé diacétylénique de formule (7) étant lui-même préparé par réaction du composé diacétylénique de formule : 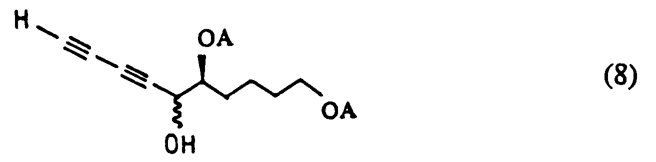 (où A a la même signification que précédemment) avec EtMgBr (où Et = éthyle)dans un solvant, en particulier le tétrahydrofuranne, sous argon, puis avec l'aldéhyde de formule (9) en solution dans un solvant :

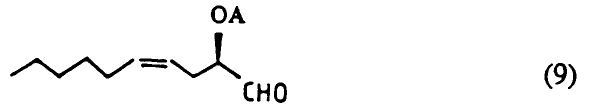

L'aldéhyde de formule (9) est connu et sa préparation est décrite dans l'article de G. Solladié et collaborateurs, Tetrahedron Asymmetry (1991), Vol 2, n° 6, pages 457-469, le schéma de la réaction étant donné page 460.

La préparation du composé diacétylénique de formule (8) se fait, avantageusement, de la façon suivante :
1) réaction de la valérolactone avec l'alcool méthylique en présence d'acide sulfurique pour obtenir le méthyl 5-hydroxypentanoate,
2) éthérification de la fonction alcool pour obtenir l'éther de formule : 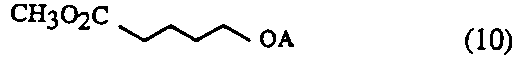
3) addition de méthyl p-tolylsulfoxyde sur l'éther de formule (10) pour obtenir le sulfoxyde de formule (avec tol = tolyle) : 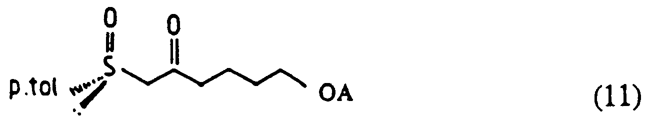 en présence de lithium diisopropyl amidure en solution dans un solvant, en particulier le tétrahydrofuranne, à une température comprise entre - 78°C et 20°C.
4) réduction du groupe carbonyle du composé de formule (11) par le diisobutyl aluminium hydrure à une température comprise entre -70 et -85°C pour obtenir le sulfoxyde de formule : 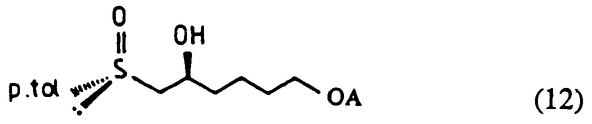
5) éthérification de la fonction alcool du sulfoxyde de formule (12) par le dérivé chloré de formule ACl, où A a la même signification que ci-dessus, en présence d'imidazole pour obtenir le sulfoxyde de formule : 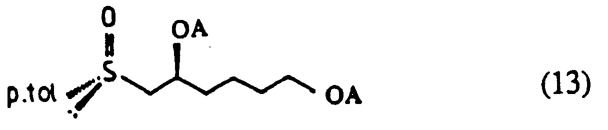
6) réaction d'acétate de sodium et d'anhydride acétique sur le composé de formule (13) pour obtenir le dérivé acétoxylé de formule (avec Ac = acétyle) : 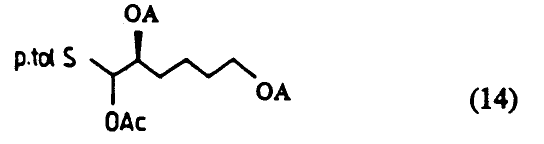
7) réaction avec du lithium tri-(sec-butyl)-borohydrure pour obtenir l'alcool de formule : 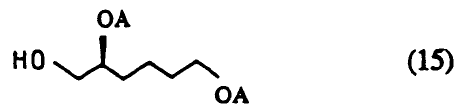
8) réaction avec du pyridinium chlorochromate pour obtenir l'aldéhyde de formule : 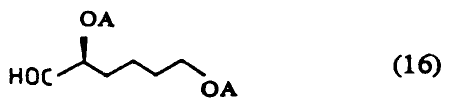
9) réaction avec le composé de formule :

   BrMg-≡-≡-H

   pour obtenir le composé diacétylénique de formule (8).

Selon l'invention, le radical A utilisé est, de préférence, le radical t-butyldiméthylsilyle (TBDMS).

Plusieurs des composés préparés comme produits intermédiaires au cours du processus de préparation sont nouveaux. C'est le cas des composés de formule 2 à 4, 7, 8 et 11 à 16 dans lesquels A est le radical t-butylméthylsilyle (TBDMS) et B est le radical phényle (Ph), du triol de formule (5) et de la lactone de formule (6).

Par conséquent, la présente invention a également pour objet, à titre de composés chimiques nouveaux utiles comme produits intermédiaires, les produits suivants (tol = tolyle, Ac = acétyle et TBDMS = t-butyldiméthylsilyle, Ph = phényle) :
a) le sulfoxyde de formule : 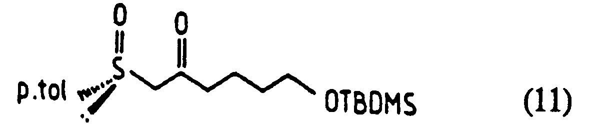
b) le sulfoxyde de formule : 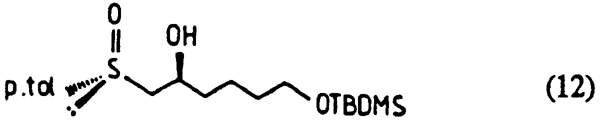
c) le sulfoxyde de formule : 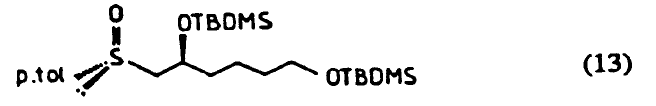
d) le dérivé acétoxylé de formule : 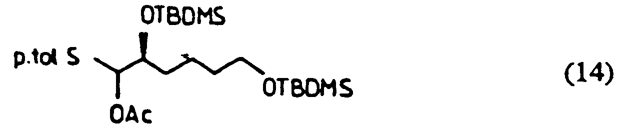
e) l'alcool de formule : 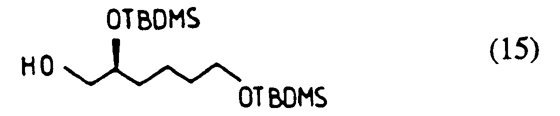
f) l'aldéhyde de formule : 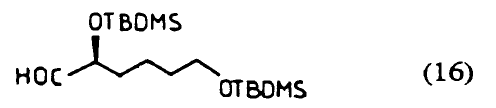
g) le composé diacétylénique de formule : 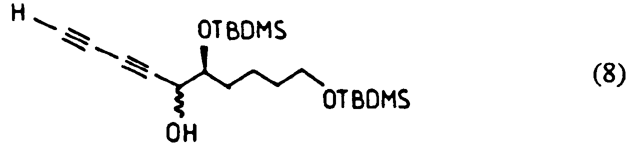
h) le composé diacétylénique de formule : 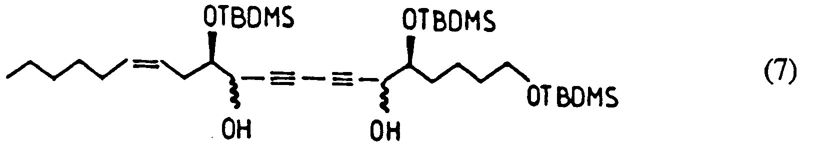
i) le dérivé de butadiène diol de formule : 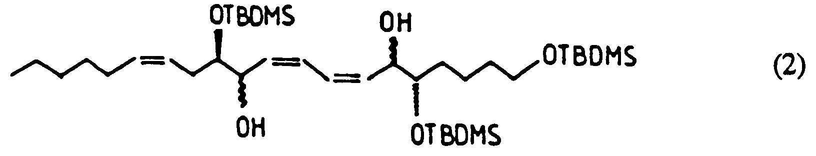
j) le dibenzoate de formule : 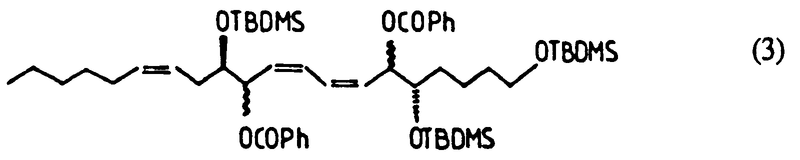
k) le triéther de formule : 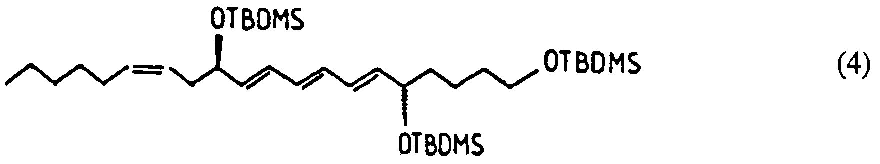
l) le triol de formule : 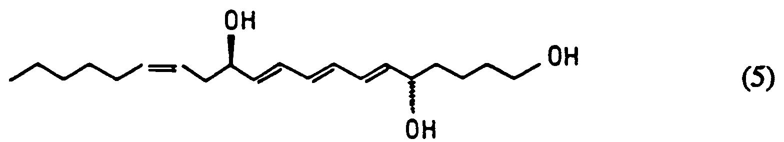
m) la lactone de formule :

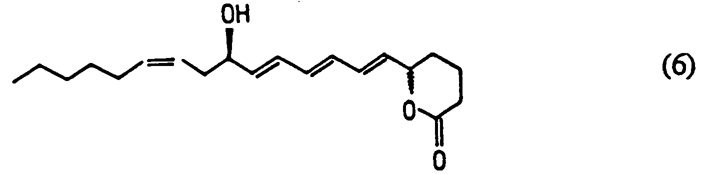

Un exemple de préparation du 6 E-LTB₄ est donnée ci-après à titre purement illustratif et non limitatif.
**1ère étape : Préparation** du **méthyl 5-hydroxypentanoate** de formule :

   H₃CO₂C-(CH₂)₃-CH₂OH (17)

   On ajoute 10 gouttes d'H₂SO₄ concentré à 10,8 g (0,108 mol.) d'alpha-valérolactone dans 200 ml de méthanol sec et le mélange est chauffé pendant 6 heures à reflux. On ajoute ensuite à 0°C, 1 g de NaHCO₃ sous agitation. Après filtration et évaporation du méthanol sous vide à 25°C, on obtient 14,2 g (0,108 mol.) du composé de formule (17) sous forme d'une huile incolore. On a vérifié que le produit obtenu a les caractéristiques spectrales décrites dans la littérature (Huckstep M. Taylor R.J.K. ; Synthesis, 1982, 881).
**2e étape : Préparation** du **méthyl 5-(t-butyldiméthylsilyloxy) pentanoate** de formule: 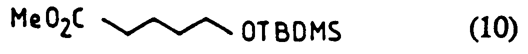
   - Me: = méthyle
   - TBDMS: = t-butyldiméthylsilyle

   A une solution de 14,2 g (0,108 mol.) de 5-hydroxypentanoate de méthyle de formule (17) dans 170 ml de diméthylformanide sec sous argon, sont ajoutés 17,8 g (0,269 mol., 2,5 équiv.) d'imidazole et 24,4 g (0,161 mol., 1,5 équiv.) de chlorure de t-butyldiméthylsilyle et le mélange est agite pendant 20 heures sous argon. On ajoute ensuite, sous forte agitation 100 ml d'une solution saturée de NH₄Cl et on extrait le mélange deux fois avec un volume équivalent d'éther. Les phases organiques sont ensuite lavées avec des solutions saturées de NH₄Cl et NaCl puis séchées sur MgSO₄, filtrées sur célite et concentrées sous vide. L'huile obtenue est ensuite purifiée par chromatographie (hexane/acétate d'éthyle 95/5) pour donner 18,0 g (0,073 mol., rendement 68%) de composé de formule (10) sous forme d'huile.
**3e étape : Préparation** de la **[(S)R]-6-(t-Butyldimethylsilyloxy)-1-p-tolylsulfinyl-2-hexanone** de formule: 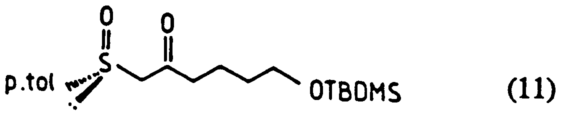
   - TBDMS: = t-butyldiméthylsilyle et tol = tolyle

   On ajoute goutte à goutte 7,20 g (0,047 mol., 2 équiv.) de (+)(R)-méthyl p-tolylsulfoxyde dissous dans 35 ml de tétrahydrofuranne (THF) à une solution de lithium diisopropylamidure (0,049 mol., 2,1 équiv.) dans 100 ml de THF sous argon, refroidie à -78°C. La solution jaune résultante est agitée pendant 1 heure à -78°C ,puis pendant 30 minutes à 0°C, et refroidie à nouveau à -78°C. L'ester de formule (10) (5,8 g, 0,023 mol., 1 équiv.), en solution dans 35 ml de THF, est alors ajouté en 30 minutes. La solution résultante est réchauffée lentement et agitée pendant 2 heures à température ambiante. Après hydrolyse avec 200 ml d'une solution saturée de NH₄Cl, le mélange est extrait deux fois par un volume égal d'éther et les phases organiques lavées avec des solutions saturées de NH₄Cl et NaCl puis séchées sur MgSO₄ et concentrées sous vide. L'huile brute est chromatographiée (hexane/acétate d'éthyle: 50/50) pour obtenir 7,04 g, (0,019 mol.) d'hexanone de formule (11) sous forme d'une huile légèrement jaune. Le rendement est de 81 %.
   Les caractéristiques de ce produit sont les suivantes :
   a)Rf = 0,48(50% acétate d'éthyle dans l'hexane)
   b)[α]²⁴D = +137,7 (c=1,6, CHCl₃) ;
   c)IR(CH₂Cl₂) 2850-2920,1700,1350,1090cm⁻¹;
   d)¹H NMR (CDCl₃)δ: 7,52 (d, J = 8,3Hz, 2H), 7,31(d, J = 8,4Hz, 2 H), 3,84 (d, J = 13,5 Hz, 1H); 3,71(d, J = 13,5 Hz, 1 H), 3,56(t, J = 6,1 Hz, 2 H) ; 2,49 (m,2H) ; 2,40 (s,3H), 1,6-1,4 (m,4H), 0,86 (S,9H), 0,02 (s,6 H) ;
   e)¹³C NMR (CDCl₃)δ: 142,13 ; 139,72 ; 130,14 ; 124,10 ; 68,12 ; 62,62 ; 44,71 ; 31,83 ; 25,96 ; 21,42 ; 19,62 ; 18,38 ; -5,4 .
**4e étape : Préparation** de **[(S)R]-2-(S)-6-(t-Butyldiméthylsilyloxy)-1-p-tolylsulfinyl-2-hexanol** de formule : 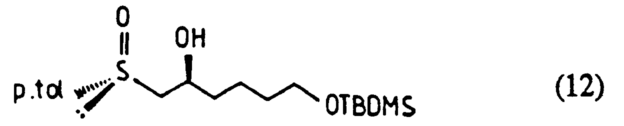
   - p-tol: = p-tolyle
   - TBDMS: = t-butyldiméthylsilyle

   On ajoute 50 ml de diisobutyl aluminium hydrure (0,05 mol., 2,7 équiv., solution 1M dans le toluène) goutte à goutte, en 2 heures, à une solution de l'hexanone de formule (11) (6,85 g, 0,018 mol., 1 équiv.) dans 170 ml de tétrahydrofuranne anhydre, à -78 °C sous argon. Le mélange est ensuite agité pendant 1,5 heure. On ajoute ensuite à -78°C, 10 ml de méthanol et 300 ml d'acétate d'éthyle. On ajoute ensuite 300 ml d'une solution saturée de tartrate de sodium à température ambiante et le mélange est agité pendant 30 minutes. La phase organique est séparée, lavée avec des solutions saturées de NH₄Cl et NaCl, séchée sur MgSO₄ et concentrée sous vide. Après purification par chromatographie (hexane/acétate d'éthyle : 50/50), on obtient le composé de formule (12) (5,5 g, 0,015 mol., rendement 80%) sous forme d'une huile jaunâtre. Un rapport de diastéréoisomères égal à 98:2 a été déterminé par RMN du ¹H du produit brut. Un rendement de 87% a été calculé en tenant compte de la quantité (0,56 g, 1,5 mmol.) d'hexanone de formule (11) qui n'a pas réagi et qui peut être recyclée.
   Les caractéristiques du produit obtenu sont les suivantes :
   a)Rf= 0,48(60% acétate d'éthyle dans l'hexane);
   b)[α]²⁴D = +123,2 (c= 1,9 CHCl₃) ;
   c)IR(CH₂Cl₂) 3650,3450-3300, 2850-2920, 1100 cm⁻¹;
   d)¹H NMR (CDCl₃)δ: diastéréoisomère principal (98%) : 7,50 (d, J = 8,2 Hz, 2H), 7,32 (d, J = 8,2 Hz, 2H), 4,65 (m,1H) 3,55 (t, J = 6,0 Hz, 2H); 2,98 (dd, J = 13,4 et 9,8Hz, 1H) 2,66 (dd, J = 13,3 et 1,6 Hz, 1 H) ; 2,41 (s, 3 H), 1,44 (m, 6 H), 0,85 (s, 9 H), 0.05 (s, 6H) ; diastéréoisomère secondaire (2%) : 2,85 (dd), 2,71 (dd) et 0,86 (s), 0,018 (s) ;
   e)¹³C NMR (CDCl₃)δ: 141,4 ; 139,6 ; 130,0 ; 123,8 ; 66,4 ; 62,9 ; 61,7 ; 36,7 ; 32,4; 25,9 ; 21,4 ; 21,35 ; 18,3 ; 5,35 ;
**5e étape : Préparation** de **[(S)R]-2(S)-2,6-Bis(t-butyldiméthylsilyloxy)-1-p-tolylsulfinyl hexane** de formule : 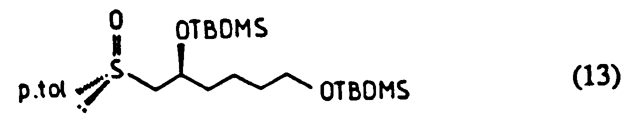
   - p-tol: = p-tolyle
   - TBDMS: = t-butyldiméthylsilyle

   On ajoute 5,5 g (14,8 mmol., 1 équiv.) du composé de formule (12) dans 60 ml de diméthylformamide sec sous argon à 2,5 g (37,9 mmol., 2,5 équiv.) d'imidazole et 3,4 g (22,5 mmol., 1,5 équiv.) de chlorure de t-butyldiméthylsilyle et le mélange est agité pendant 12 heures. On ajoute ensuite 100 ml d'une solution saturée de NH₄Cl. La phase aqueuse est extraite avec un volume égal d'éther et les phases organiques sont lavées avec une solution saturée de NaCl, séchées sur MgSO₄ et évaporées. Après purification par chromatographie (hexane/acétate d'éthyle : 80/20), on isole l'hexane de formule (13) (7,1 g, 14,6 mmol., rendement 98%) sous forme d'une huile légèrement jaune.
   Les caractéristiques de ce produit sont les suivantes :
   a)Rf = 0,76 (50% acétate d'éthyle dans l'hexane) ;
   b)[α]²⁴D = +125,0 (c = 2,0, CHCl₃) ;
   c)IR (CH₂CL₂) 2850-2920, 1100cm⁻¹ ;
   d)¹H NMR (CDCl₃)δ: 7,49 (d, J = 8,2 Hz, 2H); 7,29 (d, J = 8,2 Hz, 2H); 4,25 (m, 1H); 3,55 (t, J = 6,0 Hz, 2H) ; 2,81 (dd, J = 12,9 et 3,1 Hz, 1H) ; 2,68 (dd, J = 12,9 et 9,3 Hz, 1H) ; 2,39 (s, 3H) ; 1,6-1,4(m,6H) ; 0,92-0,85 (m, 18 H) ; 0,19-0,05 (m, 2 H) ;
   e)¹³C NMR (CDCl₃)δ: 141,6 ; 141,08 ; 129,9 ; 123,7 ; 66,9 ; 66,5 ; 62,5 ; 37,4 ; 32,8 ; 25,9 ; 29,85 ; 25,65 ; 20,65 ; 18,1 ; -3,59 ; -4,27 ; -4,72, - 5,35 ;
**6e étape : Préparation** de **2(S)-1-Acétoxy-2,6-Bis(t-butyldiméthylsilyloxy)-1-p-tolylsulfide-hexane** de formule : 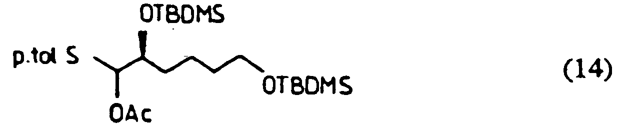
   - Ac: = acétyle
   - p-tol: = p-tolyle
   - TBDMS: = t-butyldiméthylsilyle

   On ajoute 4,7 g (57,3 mmol, 4 équiv.) d'acétate de sodium à 7,0 g (14,4 mmol., 1 équiv.) du composé de formule (13) dans 100 ml d'acide acétique anhydre, sous argon, et on chauffe à 120°C durant 22 heures. Un volume équivalent de toluène est alors ajouté et l'excès d'acide acétique anhydre est éliminé par distillation azéotropique, l'opération étant répétée 3 fois avant d'ajouter 200 ml d'éther aux résidus. On sèche sur MgSO₄, on filtre sur célite et on évapore le solvant. Après chromatographie (hexane/acétate d'éthyle : 91/9), on obtient 5,0 g (9,45 mmol., rendement 66 %) du composé de formule (14) sous forme d'une huile jaune. On a isolé 1,2 g d'un produit secondaire et on l'a identifié comme étant le sulfure dans lequel le groupe TBDMS de l'hydroxyle en position 2 a été remplacé par un groupe acétate. En tenant compte de ce produit secondaire qui peut être recyclé, le rendement de la réaction serait de 84%.
   Les caractéristiques du produit obtenu sont les suivantes :
   a)Rf = 0,66 (20% acétate d'éthyle dans l'hexane) ;
   b)[α]²⁴D = + 6,0 (c = 1,0, CHCl₃) ;
   c)IR(CH₂Cl₂) : 2850-2950, 1730,1230,1260,1110 cm⁻¹ ;
   d) ¹H NMR (CDCl₃)δ: 7,35 (dd, J = 8,2 et 6,5 Hz, 2H) ; 7,09 (dd, J = 8,2 et 1,8 Hz, 2H) ; 6,09 (d, J = 2,3Hz, 0,5H) ; 6,01 (d, J =6,4Hz, 0,5H), 3,91 (ddd, J = 7,2, 7,1 et 2,3 Hz, 0,5H) ; 3,81 (dd, J = 11,5 et 6,2 Hz, 0,5H), 3,60 (m, 2 H) ; 2,18 (d, J = 3,6 Hz, 3H) ; 2,1 (s, 3 H) ; 1,38 (m, 2H); 1,25(m 4 H), 0,89 (m, 18 H) ; 0,06 (m, 12 H) ;
   e)¹³C NMR(CDCl₃)δ: mélange de diastéréoisomères 169,6 ; 169,2 137,8 ; 137,95 ; 133,72 ; 132,89 ; 129,74 ; 129,72 ; 128,52 ; 129,3 86,72 ; 84,05 ; 74,5 ; 72,76 ; 63,04 ; 62,99 ; 33,64; 33,11 ; 33,03 32,84 ; 25,81 ; 25,97 ; 25,75 ; 22,06 ; 21,21; 21,15; 21,1 ; 20,99 ; 18,0 ; -4,5 ; -4,6 ; -5,3 ;
**7e étape** : **Préparation** de **2(S)-2,6-Bis(t-butyldiméthylsilyloxy)hexan-1-ol** de formule : 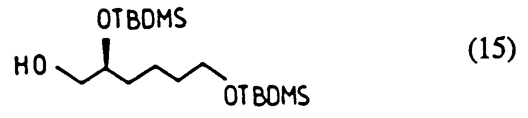
   - TBDMS: = t-butyldiméthylsilyle

   A une solution de 4,8 g (9,07 mmol., 1 équiv.) du composé de formule (14) dans 180 ml de tétrahydrofuranne (THF), refroidie à -78°C, on ajoute goutte à goutte 36 ml (solution 1M/THF, 36,0 mmol., 4 équiv.) de lithium-tri(sec-butyl)-borohydrure vendu sous la dénomination commerciale "L-SELECTRIDE" par la société "ALDRICH". A la fin de l'addition, le mélange est lentement réchauffé à température ambiante et agité pendant 1,5 heure. On refroidit à nouveau à -78°C et on ajoute doucement 20 ml de méthanol. On laisse revenir à température ambiante, on ajoute plusieurs spatules de SiO₂ et on agite encore pendant 1 heure, avant de filtrer sur célite. On évapore le solvant et on purifie par chromatographie (hexane/acétate d'éthyle : 89/11). On recueille 2,44 g (6,70 mmol.) d'alcool de formule (15) sous forme d'huile. Le rendement est de 74%.
   Le produit obtenu a les caractéristiques suivantes :
   a)Rf = 0,35 (11 % acétate d'éthyle dans l'hexane) ;
   b) IR (CHCl₃) : 3400, 2840-2905, 1450 cm⁻¹;
   c)¹H NMR (CDCl₃)δ; 3,73 (m, 1H) ; 3,60 (t, J = 6,1 Hz, 2 H); 3,56 (dd, J = 7,9 et 3,9 Hz, 1H) ; 3,43 (dd, J = 11,5 et 5,7 Hz, 1 H) ; 1,72 (large m, 1 H) ; 1,55-1,4 (m, 6 H) ; 0,89 (m, 18 H) ; 0,01 (m, 12 H) ;
   d)¹³C NMR (CDCl₃)δ: 72,89 ; 66,25 ; 62,98 ; 33,78 ; 32,97 ; 25,96, 25,85 ; 21,69 ; 18,34 ; 17,60 ; -4,40 ; -4,56 ; -5,32 ;
**8e étape : Préparation** de **2(S)-2,6-Bis(t-butyldiméthylsilyloxy)-hexan-1-al** de formule : 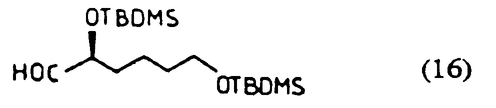
   - TBDMS: = t-butyldiméthylsilyle

   A une solution de 2,2 g (6,04 mmol., 1 équiv.) du composé de formule (15) dans 120 ml de CH₂Cl₂ sec, sous argon, en présence de tamis moléculaires 4Å, sont ajoutés 3,4 g (15,8 mmol., 2,6 équiv.) de pyridinium chlorochromate(PCC). Après 4 heures, on ajoute 200 ml d'éther et on filtre sur célite. Après évaporation du solvant et chromatographie (hexane/acétate d'éthyle : 89/11), on isole 1,88 g (5,19 mmoles) d'aldéhyde de formule (16) sous forme d'une huile légèrement jaune ; le rendement est de 86%.
   Le produit obtenu a les caractéristiques suivantes :
   a)Rf = 0,68 (11% acétate d'éthyle dans l'hexane) ;
   b)[α]²⁴D = - 21,6 (c = 0,85, CHCl₃) ;
   c)IR (CHCl₃) : 2840-2905, 1720, 1450, 1380, 1090 cm⁻¹ ;
   d)¹H NMR (CDCl₃)δ: 9,52 (d, J = 1,7 Hz, 1 H) ; 3,96 (dt, J = 5,7 et 1,6 Hz, 1 H), 3,60 (t, J = 5,9 Hz, 2H) ; 1,6-1,4 (m, 6H) ; 0,89 (m, 18 H) ; 0,02 (m, 12H) ;
   e)¹³C NMR (CDCl₃)δ : 198,5 ; 77,7 ; 62,81 ; 32,64 ; 32,46 ; 25,92; 25,73 ; 21,19 ; 18,17 ; -4,6 ; -4,9 ; -5,3.
**9e étape : Préparation** de **6(S)-6,10-Bis(t-butyldiméthylsilyloxy)-5-hydroxy-1,3-décadiyne** de formule : 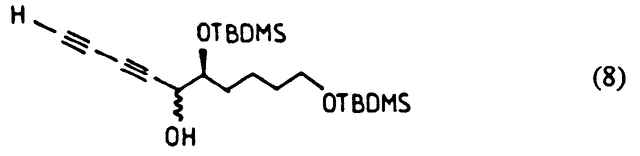
   - TBDMS: = t-butyldiméthylsilyle

   A 1,0 g (20,0 mmol, 4 équiv.) de diacétylène dans 20 ml de tétrahydrofuranne sec refroidi à -78°C, sont ajoutés 10 ml d'une solution 1M de bromure d'éthylmagnésium dans l'éther (10,0 mmol., 2 équiv.) de façon à obtenir le composé de formule :

   BrMg-≡-≡-H

   La solution est ensuite agitée pendant 1,5 heure à température ambiante. On refroidit à nouveau à -78°C et l'aldéhyde de formule (16) (1,8 g, 4,97 mmol., 1 équiv.), en solution dans 15 ml d'éther, est ajouté sur une période de 20 minutes. On agite ensuite pendant 1 heure à 0°C, on hydrolyse avec 100 ml d'une solution saturée de NH₄Cl et on extrait trois fois à l'éther. Les phases organiques sont lavées avec des solutions saturées de NH₄Cl et NaCl, séchées sur MgSO₄, concentrées sous vide et chromatographiées (hexane/acétate d'éthyle : 89/11). On obtient 1,69 g (4,10 mmol.) de dérivé diacétylénique de formule (8) sous forme d'une huile, qui noircit rapidement. Le rendement est de 82%.
   Le produit obtenu a les caractéristiques suivantes :
   a)Rf = 0,41 (11 % acétate d'éthyle dans l'hexane) ;
   b)[α]²⁴D = + 3,4 (c = 0,88, CHCl₃) ;
   c)IR (CHCl₃) : 3500 (large), 3290 (étroite), 2840-2905, 1450, 1350, 1100 cm⁻¹ ;
   d)¹H NMR (CDCl₃)δ: 4,35 (d, J = 3,5 Hz, 0,6 H) ;
      4,25 (d, J = 3,3 Hz, 0,4H) ; 3,75 (m, 1 H) ; 3,60 (dt, J = 6,2 et 2,1 Hz, 2H); 2,15 (m, 1H) ; 1,6 - 1,3 (m, 6H); 0,85 (m, 18H); 0,2 -0,0 (m, 12 H) .
   e)¹³C NMR ((CDCl₃)δ: 75,1 ; 74,8 ; 67,95 ; 67,9 ; 66,3 ; 64,9 ; 62,85 ; 33,48 ; 32,8 ; 32,4 ; 25,96 ; 25,82 ; 25,8 ; 21,64 ; 21,48 ; 18,06 .
**10e étape : Préparation** de **[4Z,2(R)]-2-(t-Butyldiméthylsilyloxy)-4-decen-1-al** de formule : 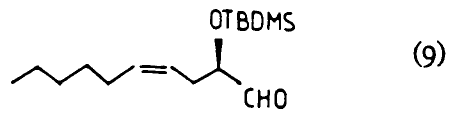
   - TBDMS: = t-butyldiméthylsilyle

   L'aldéhyde de formule (9) est préparé par le procédé décrit aux pages 461 à 465 de la publication "Tetrahedron Asymmetry, Vol. 2, n° 6, pages 457-468", selon le schéma : 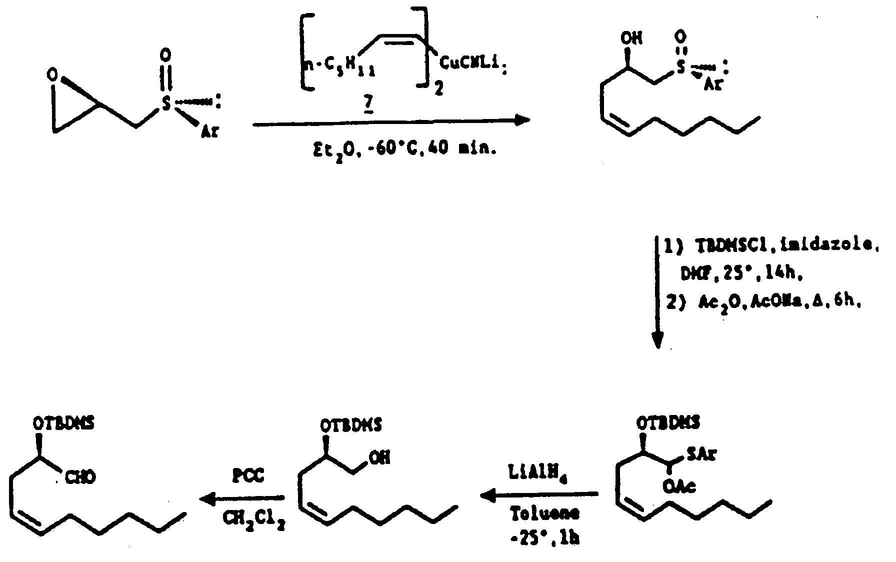 formules dans lesquelles :
   - Ar: = p-tolyle
   - Et: = éthyle
   - TBDMS: = t-butyldiméthysilyle
   - DMF: = diméthylformamide
   - Ac: = acétyle
   - PCC: = pyridinium chlorochromate
**11e étape: Préparation** de **[14Z,5(S),12(R)]-1,5,12-tris(t-butyldiméthylsilyloxy)-6,11-bis-hydroxy-14-eicosene-7,9-diyne** de formule : 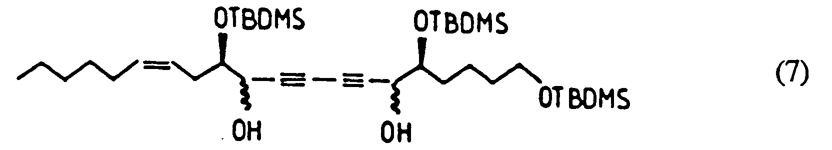
   - TBDMS: = t-butyldiméthylsilyle

   Le composé diacétylénique de formule (8) (0,58 g, 1,41 mmol., 1 équiv.) en solution dans 5 ml de tétrahydrofuranne (THF) est traité à 0°C par C₂H₅ MgBr (1M dans l'éther, 3,0 ml, 2,1 équiv.), puis est agité à température ambiante pendant 1,5 heure. On refroidit à nouveau à 0°C et on ajoute goutte à goutte l'aldéhyde de formule (9) (0,4 g, 1,41 mmol., 1 équiv.) dissous dans 2 ml de THF sur une période de 10 minutes. Le mélange est alors agité à température ambiante pendant 3 heures, hydrolysé par 10 ml d'une solution saturée de NH₄Cl et extrait trois fois à l'éther. Les phases organiques sont enfin lavées par des solutions saturées de NH₄Cl et NaCl, séchées sur MgSO₄, concentrées, et le résidu est purifié par chromatographie (hexane/acétate d'éthyle : 94/6). On recueille 0,63 g (0,90 mmol.) de composé diacétylénique de formule (7) sous forme d'huile jaune avec un rendement de 63%. Un rendement de 89% peut être calculé, si on recycle le composé diacétylénique de formule (8) (0,16 g, 0,39 mmol.) n'ayant pas réagi.
   Les caractéristiques du produit obtenu sont les suivantes :
   a)Rf = 0,34 (11% acétate d'éthyle dans l'hexane) ;
   b)¹H NMR (CDCl₃)δ: 5,50 (m,1H,H₁₅) ; 5,45 (m, 1H, H₁₄) ; 4,37 (t, J = 3,6 Hz, 1H, H₁₂) ; 4,27 (t, J = 3,2Hz, 1H, H₅) ; 3,77 (m,2H, H_{6,11}) ; 3,60 (dt, J = 6,1 et 1,3Hz, 2H, H₁) ; 2,38 (m,2H,H₁₃) ; 2,03 (m,2H, H₁₆) ; 1,60 - 1,45 (m, 6H) ; 1,30 (m, 6H) ; 0,90 (m, 30H) ; 0,10 (m, 18H) ;
**12e étape : Préparation** de **(7Z, 9Z, 14Z, 5(S), 12(R)]-1,5,12-tris(t-butyldiméthylsilyloxy)-6,11-bis-hydroxy-7,9,14-eicosa-triene** de formule : 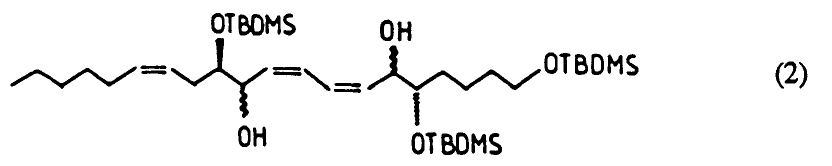
   - TBDMS: = t-butyldiméthylsilyle

   On prépare tout d'abord du zinc activé selon la méthode décrite dans Bolland W et collaborateurs ; Helv; Chim. Acta, 1987, 70,1025 . Du zinc en poudre (6,3 g, 96,9 mmol.) dans 40 ml d'eau distillée est agité pendant 15 minutes sous argon. On ajoute alors de l'acétate de cuivre Cu(OAc)₂ (0,57 g, 3,13 mmol.) et on agite à nouveau pendant 15 minutes sous argon. AgNO₃ (0,63 g, 3,70 mmol) est ensuite ajouté et le mélange est agité pendant 30 minutes. On filtre et on lave le zinc activé successivement par de l'eau distillée, du méthanol, de l'acétone et de l'éther. Le zinc activé est ensuite transféré dans un ballon contenant 25 ml d'un mélange (méthanol/H₂O :1/1) sous argon. On ajoute le composé diacétylénique de formule (7) dissous dans 4 ml de méthanol. La réaction est suivie par chromatographie sur couche mince. Après 32 heures sous agitation, on ajoute 50 ml de méthanol, on filtre et on lave soigneusement le zinc avec du méthanol. Après évaporation du méthanol, le résidu est repris par de l'éther et séché sur MgSO₄.Après évaporation du solvant, le produit est purifié par chromatographie (hexane/acétate d'éthyle : 91/9). Le triène de formule (2) obtenu (0,50 g, 0,72 mmol) se présente sous forme d'huile et est un mélange de deux diastéréoisomères correspondant aux deux OH en position 6 et 11 dans le rapport 1/1,3. Le rendement est de 79%.
   Les diastéréoisomères obtenues ont les caractéristiques suivantes :
   a)Rf = 0,50 (secondaire) et 0,26 (principal) (14% acétate d'éthyle dans l'hexane) ;
   b)[α]²⁴D = +6,4 (c = 1,7, CHCl₃) ;
   c)IR (CHCl₃) : 3550, 2860 - 2905, 1450, 1190 cm⁻¹ ;
   d)¹H NMR (CDCl₃)δ: diastéréoisomère secondaire : 6,35 (m, 2 H, H_{8,9}, d avec J = 11,2 Hz après irradiation de m à 5,45 ppm) ; 5,60-5,30 (m, 4 H, H_{7,10,14,15}) ; 4,56 (m, 1 H, H₁₁) ; 4,41 (dd, J = 3,7 et 8,6 Hz, 1 H, H₆) ; 3,74 (m, 2 H, H_{5,12}) ; 3,58 (m, 2 H, H₁) ; 2,20 (m, 2 H, H₁₃) ; 2,00 (m, 2H, H₁₆) 1,48 (m, 6 H), 1,26 (m, 6 H), 0,89 (m, 30 H), 0,05 (m, 18H); diastéréoisomère principal : 6,35 (m, 2 H, H_{8,9}) ; 5,60-5,30 (m, 4H, H_{7,10,14,15}) ; 4,53 (dd, J = 8,9 et 3,2 Hz, 1H, H₁₁) ; 4,42 (m, 1 H, H₆) ; 3,74 (m, 1 H, H₁₂) ; 3,59 (m, 3H, H_{1,5}) ; 2,50-2,20 (m, 2H, H₁₃) ; 2,00 (q, J = 7,2 Hz, 2 H, H₁₆), 1,50 - 1,25 (m, 12 H) ; 0,89 (m, 30 H) ; 0,05 (m, 18 H).
**13e étape : Préparation** de **[7Z,9Z,14Z,5(S),12(R)]-1,5,12-tris(t-butyldiméthylsilyloxy)-6,11-bis-benzoxy-7,9,14-eicosa-triene** de formule : 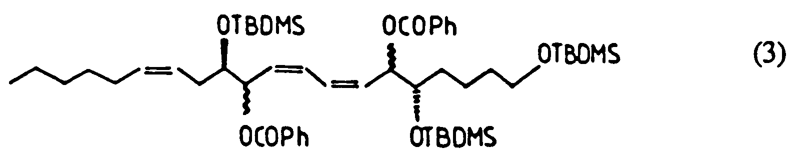
   - TBDMS: = t-butyldiméthylsilyle
   - Ph: = phényle

   Au mélange des deux diastéréoisomères de formule (2) (0,502 g, 0,72 mmol., 1 équiv.) en solution dans 12 ml de pyridine anhydre et sous argon, sont ajoutés 0,21 ml de chlorure de benzoyle (1,8 mmol., 2,25 équiv.). Après 12 heures de réaction, on ajoute 100 ml d'éther, on lave deux fois avec un volume égal de H₂SO₄ 5%, NaHCO₃ saturé et NaCl saturé respectivement. La phase organique est séchée sur MgSO₄ et le solvant évaporé. Après chromatographie (hexane/acétate d'éthyle : 94/6), on isole 0,56 g (0,62 mmol.) de dibenzoate de formule (3) sous forme de deux diastéréoisomères dans le rapport 1/1,04. Le produit se présente sous forme d'huile. Le rendement est de 86%. Les deux diastéréoisomères peuvent être séparés par chromatographie.
   Les diastéréoisomères obtenus ont les caractéristiques suivantes :
   a)Rf = 0,34 (secondaire) et 0,30 (principal) (6% acétate d'éthyle dans l'hexane) ;
   b)[α]²⁴D =-22,2(c = 1,2 CHCl₃) ;
   c)IR (CHCl₃) : 2840-2900, 1700, 1450, 1250, 1100 cm⁻¹ ;
   d)¹H NMR (CDCl₃)δ: distéréoisomère secondaire : 8,1 (m 4H); 7,5 (m, 6H); 6,68 (m, 2H); 6,0 - 5,60 (m, 4H); 5,45 (m, 2H); 4,00 (m, 2H); 3,55 (t, J = 6,0 Hz, 2H); 2,24 (m, 2 H) 1,90 (t, J = 6,5 Hz, 2H) ; 1,50 (m, 6H) ; 1,20 (m, 6 H) ; 0,89 (m,30 H) ; 0,05 (m, 18H) ; diastéréoisomère principal : 8,1 (m, 4 H) ; 7,49 (m, 6H) ; 6,86 (dd, J = 11,6 et 9,6 Hz, 1H) ; 6,71 (m, 1H) ; 5,95 (dd, J = 11,2 et 3,5 Hz, 2 H); 5,75 (m, 2H); 5,45 (m, 2 H) ; 4,00 (m, 2H) ; 3,58 (m, 2H) ; 2,25 (m, 2H) ; 2,00 (m, 2H) ; 1,50 (m, 6H) ; 1,28 (m, 6H) ; 0,92 (m, 30H); 0,05 (m, 18 H).
**14e étape : Préparation** de **[6E,8E,10E,14Z,5(S),12(R)]-1,5,12-tris(t-butyldiméthylsilyloxy)-6,8,10,14-eicosa-tetraène** de formule : 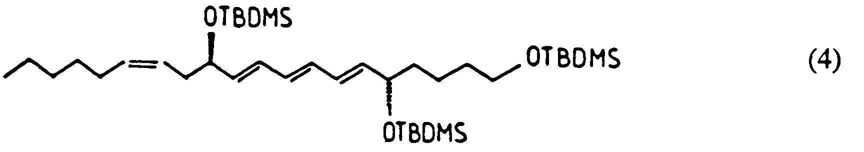
   - TBDMS: = t-butyldiméthylsilyle

   Une suspension de "Ti(0)" a été préparée selon le procédé décrit dans Walborsky et al, J. Amer. Chem. Soc., 1982, 104, 5807. On ajoute lentement 15 ml de tétrahydrofuranne (THF) anhydre à TiCl₃ (0,50 g, 3,2 mmol., 2 équiv.) dans un ballon très sec, sous argon. LiAlH₄ (1,6 ml d'une solution 1M dans l'éther, 1,6 mmol., 1 équiv.) est alors ajouté doucement. Le mélange devient rapidement noir. La suite de la réaction, y compris la chromatographie du produit, est effectuée en absence de lumière UV dans un laboratoire à lumière jaune. Après 30 minutes, 2,6 ml de cette suspension de "Ti(0)" (approximativement 0,57 mmol., 6 équiv.) sont transférés dans un ballon équipé d'un réfrigérant et sous argon. Le dibenzoate de formule (3), sous forme de mélange de diastéréoisomères (0,087 g, 0,096 mmol., 1 équiv.) dans 1 ml de THF, est ensuite ajouté à la seringue à la suspension de "Ti(0)". Le mélange réactionnel est alors immergé dans un bain chauffé à 65°C. Après 20 minutes, la chromatographie en couche mince montre que la réaction est terminée. On refroidit alors à 0°C, on ajoute successivement H₂O (1ml), HCl 0,75% (10 ml) et on extrait trois fois avec 100 ml d'éther. Les phases organiques sont successivement lavées avec HCl 0,75%, NaHCO₃ saturé, NaCl saturé, puis séchée sur MgSO₄ et évaporées. La RMN du produit brut indique qu'il est pratiquement pur. Après chromatographie (hexane/acétate d'éthyle : 96/4), on isole 0,063 g (0,094 mmol.) de triène de formule (4) sous forme d'huile. Le rendement est de 99%.
   Le produit obtenu a les caractéristiques suivantes :
   a)Rf = 0,71 (6% acétate d'éthyle dans l'hexane) ;
   b)[α]²⁴D = + 13,1 (c = 0,87, CHCl₃) ;
   c)IR (CHCl₃) : 2840-2905, 1625, 1430, 1380, 1275 cm⁻¹ ;
   d)¹H NMR (CDCl₃)δ: 6,15 (m, 4 H, H_{7,8,9,10} après découplage de m à 5,69 ppm) ; 5,69 (m, 2H, H_{6,11}, d avec J = 15,5 Hz après découplage de m à 4,15 ppm), 5,47 (m, 1 H, H₁₅, d avec J = 11 Hz après découplage de m à 2,0 ppm), 5,34 (m, 1 H, H₁₄, d avec J = 11 Hz après découplage de m à 2,3 ppm), 4,15 (q, J = 7,0 Hz, 2 H, H ₅, 12),3,59 (t, J = 3,2 Hz, 2 H,H₁), 2,28 (m, 2H, H₁₃), 2,00 (m, 2 H, H ₁₆); 1,50 (m, 6 H) ; 1,30 (m, 6H) ; 0,90 (m, 30 H) ; 0,06 (m, 18H).
   e)¹³C NMR (CDCl₃)δ: 137,26 ; 136,71; 131,91; 131,73; 129,35; 129,26; 125,51; 125,12; 73,2; 63,14; 38,2; 36,4; 32,8; 31,55; 30,32; 29,31; 27,44; 25,97; 25,90; 22,59; 21,58; 18,36; 18,27; 18,24; 14,09; -4,28; -4,40; -4,75; -4,79; -5,28.
**15e étape : Préparation** de**[6E,8E,10E,14Z,5(S),12(R)]-1,5,12-tris-hydroxy-6,8,10,14-eicosa-tetraène** de formule : 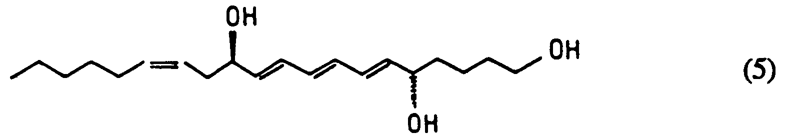
   Une solution 1M de fluorure de tétra-n-butylammonium dans le tétrahydrofuranne (THF) (0,56 ml, 6 équiv.) est ajoutée à 0°C au triène de formule (4) (0,063 g, 0,094 mmol., 1 équiv.) dans 5 ml de THF. La solution est ensuite agitée pendant 3 heures à température ambiante puis diluée par 50 ml d'éther. La phase organique est alors lavée avec une solution saturée de NaCl et la phase aqueuse est extraite deux fois avec de l'éther. Après séchage sur MgSO4 et évaporation des solvants, le produit est chromatographié (hexane/acétate d'éthyle : 25/75). On obtient 0,030 g (0,094 mmol.) de triol de formule (5) soit un rendement de 99%. Le produit est sous forme d'huile.
   Les caractéristiques du produit obtenu sont les suivantes :
   a)Rf = 0,25 (75% acétate d'éthyle dans l'hexane) ;
   b)[α]²⁴D = + 12,4(c = 0,95, CHCl₃) ;
   c)¹H NMR (CDCl₃)δ: 6,19(m, 4H, H_{7,8,9,10}, s après découplage de m à 5,71 ppm) ; 5,71 (dd, J = 14,6 et 5,9 Hz, 2 H, H_{6,11}) 5,54 (m, 1H, H₁₅, d avec J = 10,6 Hz après découplage de m à 2,1 ppm), 5,37(m, 1H, H₁₄, d avec J = 10,6 Hz après découplage de m à 2,3 ppm), 4,17 (m, 2H, H_{5,12}), 3,62 (t, J = 6,2 Hz, 2H, H₁), 2,30(m, 2H, H₁₃), 2,15 (m, 2H, H _{OH}), 2,10(m, 2H, H₁₆) 1,55 (m, 4H), 1,28 (m, 8 H), 0,90 (t, J = 6,8 Hz, 3 H) ;
   d)¹³C NMR (CDCl₃)δ: 137,64; 136,64; 134,48; 132,97; 132,84; 131,05; 130,9; 124,8; 73,17; 72,6; 63,33; 37,5; 36,01; 32,12; 32,17; 29,95; 28,09; 23,23; 22,26; 14,74.;
**16e étape : Préparation** de la **[6E,8E,10E,14Z,5(S),12(R)]-12-hydroxy-6,8,10,14-eicosa-tetraène-(1-5)-lactone** de formule : 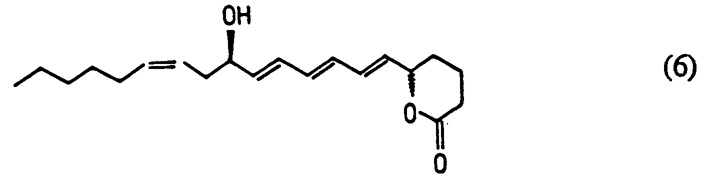
   Le triol de formule (5) (0,01 g, 0,03 mmol., 1,25 équiv.) dans 1 ml de benzène est ajouté à RuCl₂ (PPh₃)₂ préparé selon Tomioka et al, Tetrahedron Letters, 1981, 22, 1605 (0,024 g, 0,025 mmol., 1 équiv.) (avec Ph = phényle) sous argon. Après 2 heures de réaction, on ajoute 25 ml d'éther et on filtre le mélange sur silice. La purification par chromatographie en couche mince préparative (hexane/acétate d'éthyle: 50/50) conduit à 7,1 mg (0,020 mmol.) de lactone de formule (6). Le produit est sous forme d'huile. Le rendement est de 70%.
   Les caractéristiques du produit obtenu sont les suivantes :
   a)Rf = 0,34 (50% acétate d'éthyle dans l'hexane) ;
   b)[α]²⁴ D = + 8,03 (c = 0,85 CHCl₃) ;
   c))IR (CHCl₃) : 3550, 2860-2910, 1715, 1450, 1100 cm⁻¹ ;
   d)¹H NMR (CDCl₃)δ: 6,39 (m,1 H, H ₇), 6,25 (m, 3 H, H_{8,9,10,} s après découplage de m à 5,75 ppm) ; 5,80 (m, 1H, H₁₁), 5,69 (dd, J = 15,0 et 6,2 Hz, 1 H, H₆) ; 5,55 (m, 1 H, H₁₅, d avec J = 11,0 Hz après découplage de m à 2,0 ppm) ; 5,40 (m, 1 H, H₁₄, d avec J = 11,0 Hz après découplage de m à 2,35 ppm) ; 4,88 (m, 1 H, H₅) ; 4,24 (q, J = 5,7 Hz, 1 H, H₁₂) ; 2,55 (m, 2H, H₂) ; 2,32 (t, J = 6,8 Hz, 2H, H₁₃) ; 2,05 (m, 2H, H₁₆) ;1,95 (m, 2 H, H₄) ; 1,55 (m, 2H) ; 1,25 (m, 6 H) ; 0,90 (t, J = 7,0 Hz, 3H).
**17e étape : Préparation** du **leucotriène B**_{**4**} **6-trans** de formule : 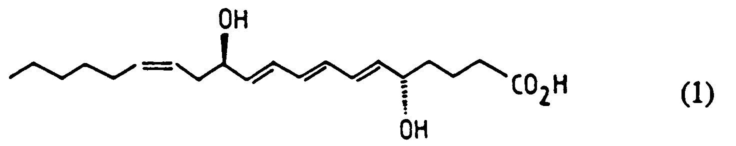
   A une solution de la lactone de formule (6) (14,8 mg, 0,046 mmol., 1 équiv.) dans 1 ml de tétrahydrofuranne est ajoutée sous argon et à -10°C, une solution 0,35M dans l'eau de LiOH (0,15 ml, 0,05 mmol., 1,1 équiv.). La réaction est terminée en 20 minutes. Après deux extractions avec CH₂Cl₂, lavage avec une solution saturée en NaCl, séchage sur MgSO₄ et évaporation des solvants, le produit est purifié par chromatographie en couche mince préparative (éthanol/acétate d'éthyle : 11/89). On obtient 7,0 mg (0,021 mmole) de LTB₄ 6-trans, qui se présente sous forme d'un solide blanc. Le rendement est de 45%.
   Le produit obtenu a les caractéristiques suivantes :
   a)Rf = 0,40 (11% d'éthanol dans l'acétate d'éthyle) ;
   b)[α]²⁴ D = + 11,2 (c = 2,0, CHCl₃) ;
   c)IR (CH₂Cl₂) : 3600-3200 (large), 3024, 3014, 2860, 1710 (étroite), 1456, 1378, 1242, 1232, 1092 cm⁻¹ ;
   d)UV I max (méthanol) : 256,5, 266,0, 277,0, (abs 0,675, 0,896 et 0,712 respectivement ), (valeurs données dans COREY E.J. et al dans Tetrahedron Letters [1981], 22, 1587 : (méthanol) 258, 268, 280) ;
   e)¹ H NMR (CDCl₃)δ: 6, 27 (m, 1H, H₇); 6,21 (m, 3 H, H_{8,9,10}); 5,74 (m, 2 H, H_{6,11}, dd avec J = 14,6 et 7,7 Hz après découplage à 4,19 ppm); 5,58 (m, 1H, H₁₅, d avec J = 11,0 Hz après découplage de m à 2,1 ppm) ; 5,34 (m, 1 H, H_{14,} dd avec J = 10,6 Hz après découplage de m à 2,35 ppm) ; 4,19 (m, 1 H, H_{5,12}) ; 2,35 (m, 4 H, H _{2,13}) ; 2,10 (m,2H, H₁₆) ; 1,65 (m, 4H) ; 1,36 (m, 6 H) ; 0,90 (t, J = 7 Hz, 3 H) ;

## Revendications

1. Procédé de préparation de leucotriène B₄ (LTB₄) sous forme 6-trans (6E) ayant pour formule : 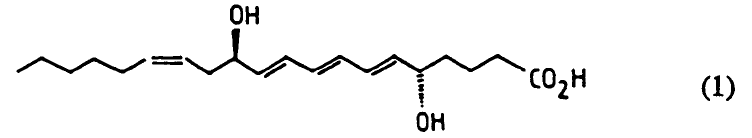 caractérisé par le fait qu'on prépare un butadiène diol de formule : 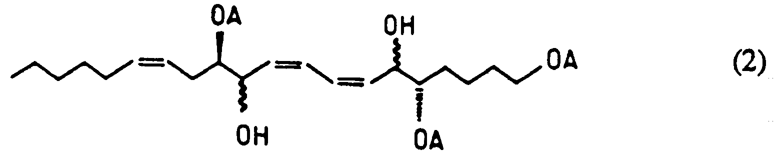 dans lequel A est un groupe protecteur silylé ; on estérifie les groupes hydroxyle pour obtenir un groupe OCOB, B étant un radical phényle ou phényle substitué par un radical alkyle ou alcoxy en C₁-C₆ pour obtenir le diester de formule : 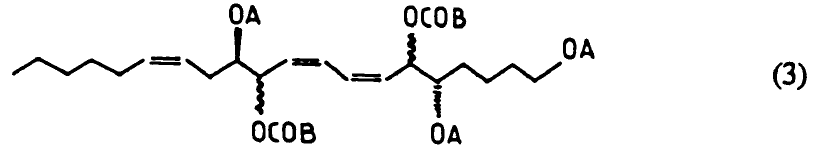 et on soumet ce diester à une élimination réductrice à l'aide de titane basse valence [Ti(0)] ou d'un amalgame de métal alcalin pour obtenir le triéther 6-trans de formule : 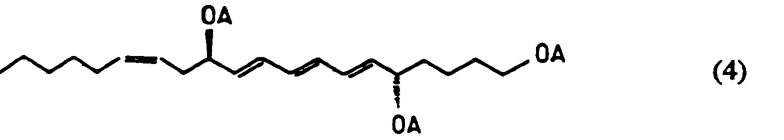 dont on transforme ensuite, les groupes -OA en positions 5 et 12 en groupes -OH et le groupe -CH₂OA en position 1 en groupe COOH, pour obtenir le LTB₄ 6-trans .

2. Procédé selon la revendication 1, caractérisé par le fait que A est un groupe t-butyldiméthylsilyle.

3. Procédé selon l'une des revendications 1 ou 2, caractérisé par le fait que B est un radical phényle.

4. Procédé selon l'une des revendications 1 à 3, caractérisé par le fait que l'élimination réductrice à l'aide de titane basse valence est effectuée dans un solvant, à une température comprise entre 50 et 70°C.

5. Procédé selon la revendication 4, caractérisé par le fait que le solvant est le tétrahydrofuranne.

6. Procédé selon l'une des revendications 1 à 5, caractérisé par le fait le triéther de formule (4) est transformé en 6 E-LTB₄ par les étapes suivantes :
a) réaction du triéther 6-trans de formule (4) avec une solution de fluorure de tétra-n-butylammonium dans un solvant, à une température comprise entre -10 et +20°C pour obtenir le triol de formule : 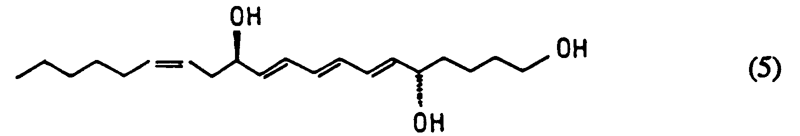
b) réaction du triol de formule (5) avec RuCl₂(PPh₃)₂ (avec Ph = phényl), sous argon, à une température comprise entre 10 et 30°C, pour obtenir la lactone de formule : 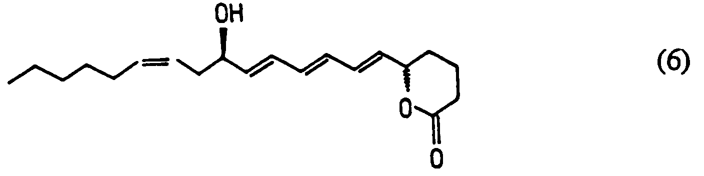 et
c) traitement de la lactone de formule (6) par LiOH en solution dans un solvant à une température inférieure à 0°C.

7. Procédé selon la revendication 6, caractérisé par le fait que le solvant utilisé aux étapes a et/ou c de la revendication 6 est le tétrahydrofuranne.

8. Procédé selon l'une des revendications 1 à 7, caractérisé par le fait que le butadiène diol de formule (2) est préparé par hydrogénation du composé diacétylènique de formule : 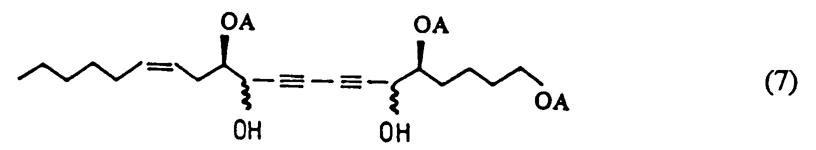 où A a la même signification que dans la revendication 1, sous argon, en présence de Zn activé en solution dans un mélange eau/méthanol, le composé diacétylènique de formule (7) étant lui-même préparé par réaction du composé diacétylénique de formule : 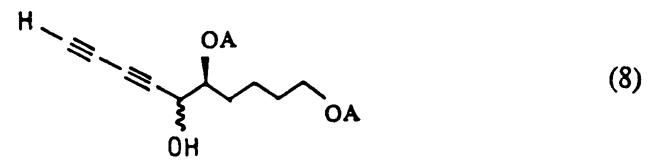 où A a la même signification que dans la revendication 1, avec C₂H₅MgBr dans un solvant, sous argon, puis avec l'aldéhyde de formule (9) en solution dans un solvant :

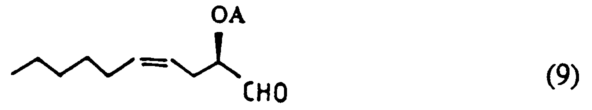

9. Procédé selon la revendication 8, caractérisé par le fait que le solvant est le tétrahydrofuranne.

10. Procédé selon l'une des revendications 8 ou 9, caractérisé par le fait que le composé diacétylènique de formule (8) est préparé par les étapes suivantes :
a) réaction de la valérolactone avec l'alcool méthylique en présence d'acide sulfurique pour obtenir le méthyl 5-hydroxypentanoate ;
b) éthérification de la fonction alcool pour obtenir l'éther de formule : 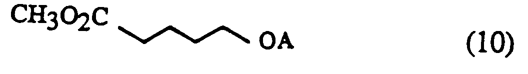 où A a la même signification qu'à la revendication 1 ;
c) addition de méthyl p-tolylsulfoxyde sur l'éther de formule (10) pour obtenir le sulfoxyde de formule (11) (avec tol = tolyle) : 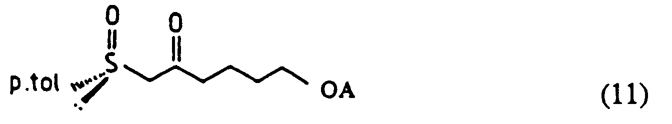 en présence de lithium diisopropyl amidure en solution dans un solvant, à une température comprise entre -78 et 20°C ;
d) réduction du groupe carbonyle du composé de formule (11) par le diisobutyl aluminium hydrure à une température comprise entre -70 et -85°C pour obtenir le sulfoxyde de formule : 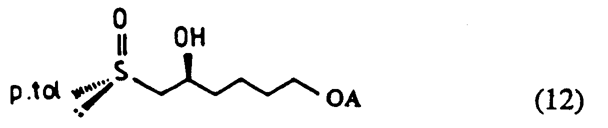
e) éthérification de la fonction alcool du sulfoxyde de formule (12) par le dérivé chloré de formule ACl, où A a la même signification que dans la revendication 1, en présence d'imidazole pour obtenir le sulfoxyde de formule : 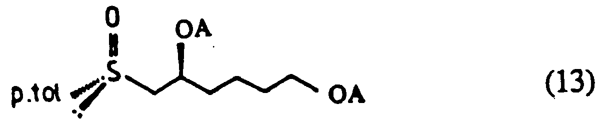
f) réaction d'acétate de sodium et d'anhydride acétique sur le sulfoxyde (13) pour obtenir le dérivé acétoxylé de formule (avec Ac = acétyle) : 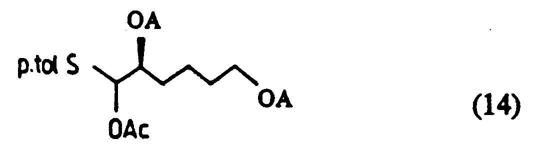
g) réaction avec du lithium tri-(sec-butyl)-borohydrure pour obtenir l'alcool de formule : 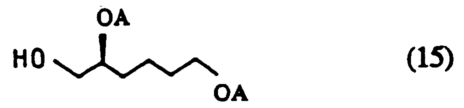
h) réaction avec le pyridinium chlorochromate pour obtenir l'aldéhyde de formule : 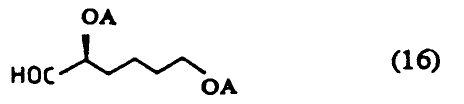
i) réaction avec le composé de formule :
BrMg-≡-≡-H
pour obtenir le composé diacétylénique de formule (8).

11. Sulfoxyde de formule : 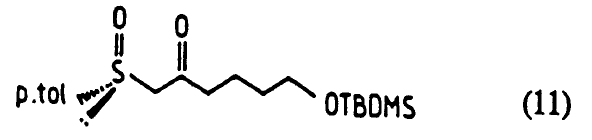 formule dans laquelle TBDMS représente le radical t-butyldiméthylsilyle et tol le radical tolyle.

12. Sulfoxyde de formule : 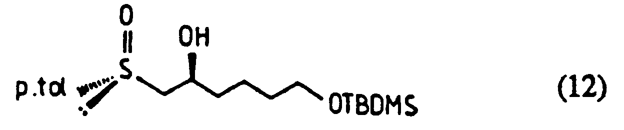 formule dans laquelle tol et TBDMS ont les significations données à la revendication 11.

13. Sulfoxyde de formule : 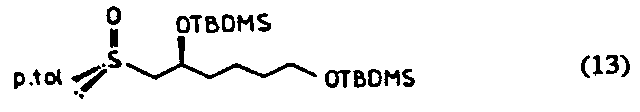 formule dans laquelle tol et TBDMS ont les significations données à la revendication 11.

14. Dérivé acétoxylé de formule : 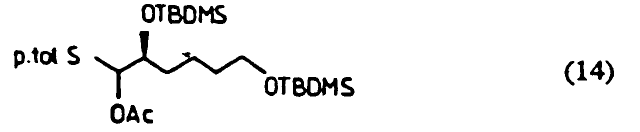 formule dans laquelle TBDMS et tol ont les significations données à la revendication 11 et Ac représente le radical acétyle.

15. Alcool de formule : 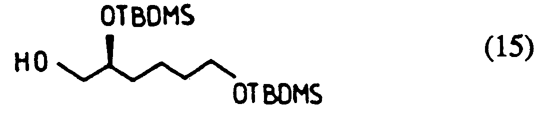 formule dans laquelle TBDMS représente le radical t-butyldiméthyl-silyle.

16. Aldéhyde de formule : 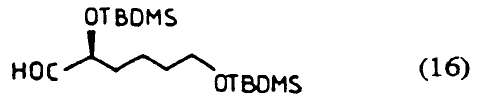 formule dans laquelle TBDMS représente le radical t-butyldiméthylsilyle.

17. Composé diacétylénique de formule : 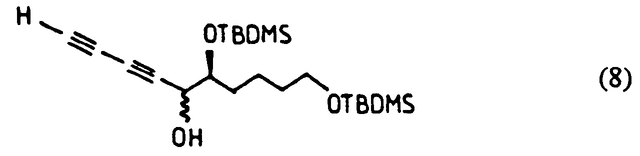 formule dans laquelle TBDMS représente le radical t-butyldiméthylsilyle.

18. Composé diacétylénique de formule : 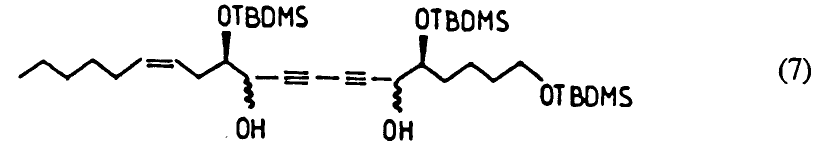 formule dans laquelle TBDMS représente le radical t-butyldiméthylsilyle.

19. Butadiène diol de formule : 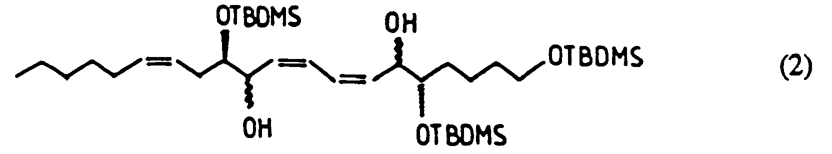 formule dans laquelle TBDMS représente le radical t-butyldiméthylsilyle.

20. Dibenzoate de formule: 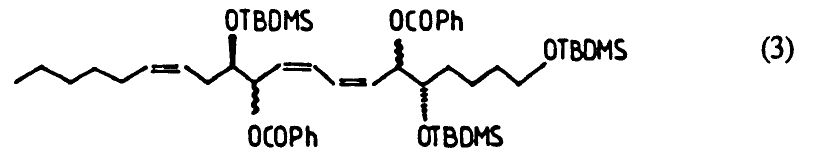 formule dans laquelle TBDMS représente le radical t-butyldiméthylsilyle et Ph représente un radical phényle.

21. Triéther de formule : 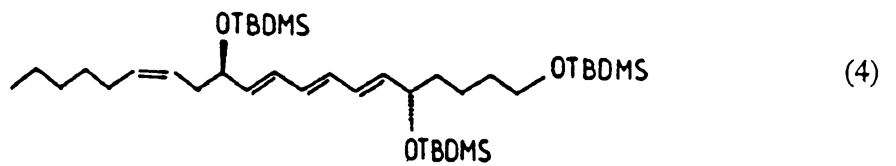 formule dans laquelle TBDMS représente le radical t-butyldiméthylsilyle.

22. Triol de formule :

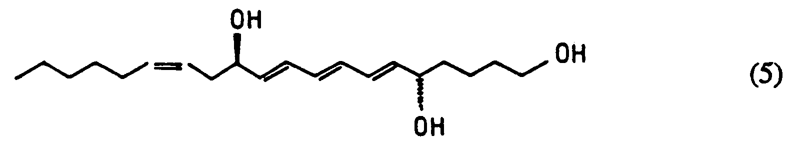

23. Lactone de formule :

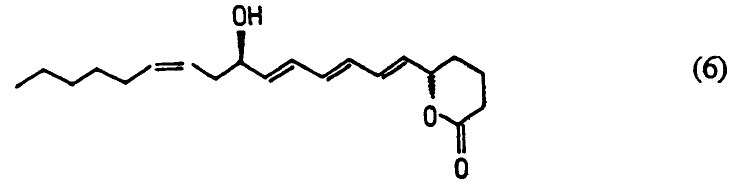

## Claims

1. Process for the preparation of leukotriene B₄ (LTB₄) in the 6-trans (6E) form, having the formula: 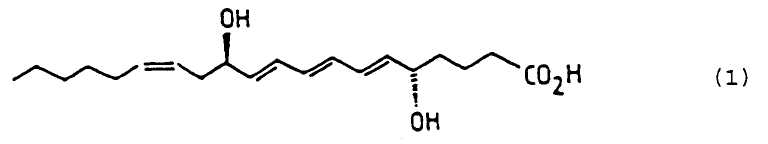 characterised in that a butadienediol of formula: 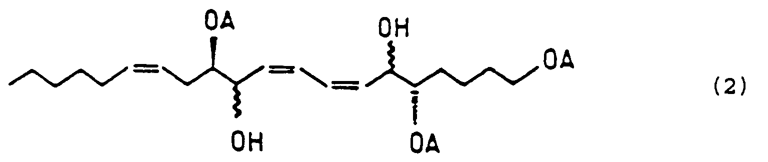 in which A is a silylated protective group, is prepared; the hydroxyl groups are esterified to produce a group OCOB, B being a phenyl radical or a phenyl radical substituted by a C₁-C₆ alkyl or alkoxy radical, to produce the diester of formula: 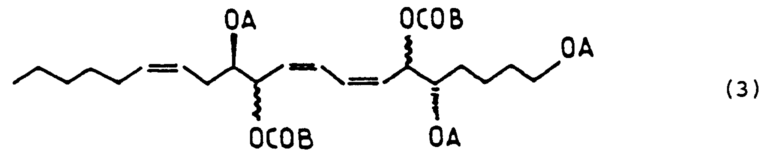 and this diester is subjected to a reductive elimination using low valency titanium [Ti(0)] or an alkali metal amalgam to produce the 6-trans triether of formula: 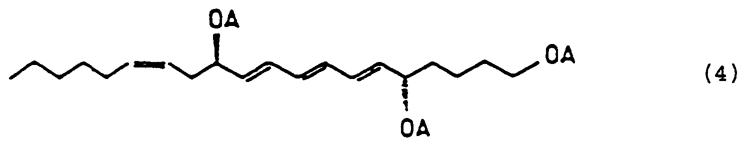 the groups -OA in positions 5 and 12 of which are then converted to -OH groups, and the group -CH₂OA in position 1 of which is then converted to a -COOH group, to produce 6-trans-LTB₄.

2. Process according to Claim 1, characterised in that A is a t-butyldimethylsilyl group.

3. Process according to either of Claims 1 or 2, characterised in that B is a phenyl radical.

4. Process according to one of Claims 1 to 3, characterised in that the reductive elimination with low valency titanium is carried out in a solvent, at a temperature of between 50 and 70°C.

5. Process according to Claim 4, characterised in that the solvent is tetrahydrofuran.

6. Process according to one of Claims 1 to 5, characterised in that the triether of formula (4) is converted to (6E)-LTB₄ by the following stages:
a) reaction of the 6-trans triether of formula (4) with a tetra-n-butylammonium fluoride solution in a solvent, at a temperature between -10° and +20°C, to produce the triol of formula: 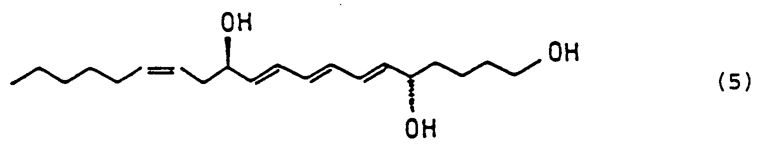
b) reaction of the triol of formula (5) with RuCl₂(PPh₃)₂ (with Ph = phenyl), under argon, at a temperature between 10 and 30°C to produce the lactone of formula: 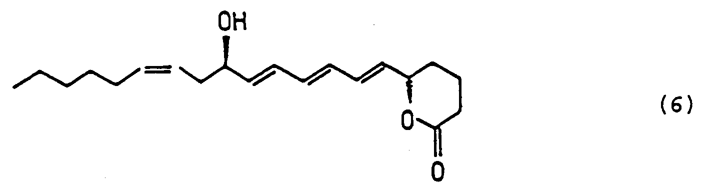 and
c) treatment of the lactone of formula (6) with LiOH in solution in a solvent at a temperature of less than 0°C.

7. Process according to Claim 6, characterised in that the solvent used in Stages a and/or c of Claim 6 is tetrahydrofuran.

8. Process according to one of Claims 1 to 7, characterised in that the butadienediol of formula (2) is prepared by hydrogenation of the diacetylene compound of formula: 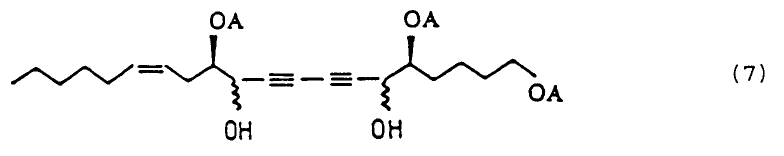 where A has the same meaning as in Claim 1, under argon, in the presence of activated Zn in solution in a water/methanol mixture, the diacetylene compound of formula (7) being itself prepared by reaction of the diacetylene compound of formula: 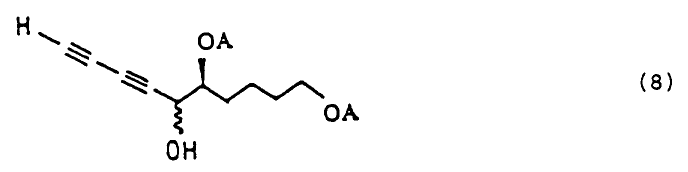 where A has the same meaning as in Claim 1, with C₂H₅MgBr in a solvent, under argon, and then with the aldehyde of formula (9) in solution in a solvent:

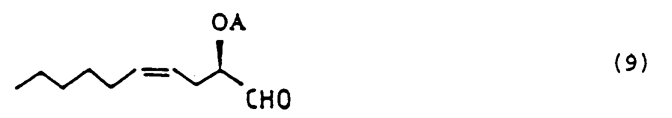

9. Process according to Claim 8, characterised in that the solvent is tetrahydrofuran.

10. Process according to either of Claims 8 or 9, characterised in that the diacetylene compound of formula (8) is prepared by the following stages:
a) reaction of valerolactone with methyl alcohol in the presence of sulphuric acid to produce methyl 5-hydroxypentanoate;
b) etherification of the alcohol functional group to produce the ether of formula: 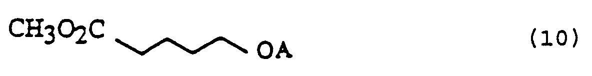 where A has the same meaning as in Claim 1;
c) addition of methyl p-tolyl sulphoxide to the ether of formula (10) to produce the sulphoxide of formula (11) (with tol = tolyl): 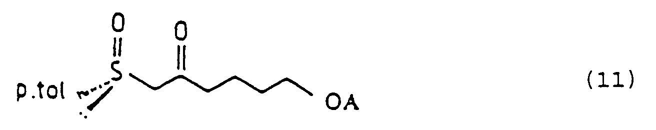 in the presence of lithium diisopropylamide in solution in a solvent at a temperature between -78°C and 20°C;
d) reduction of the carbonyl group of the compound of the formula (11) by diisobutylaluminium hydride at a temperature between -70 and -85°C to produce the sulphoxide of formula: 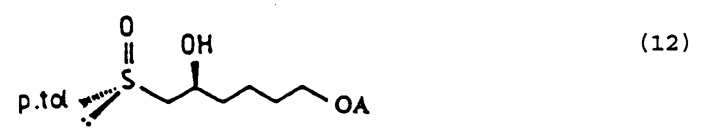
e) etherification of the alcohol functional group of the sulphoxide of formula (12) by the chlorinated derivative of formula ACl where A has the same meaning as in Claim 1, in the presence of imidazole to produce the sulphoxide of formula: 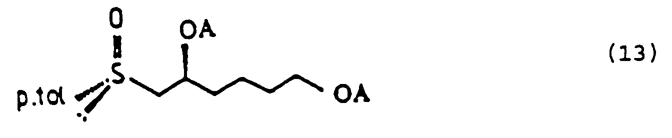
f) reaction of sodium acetate and acetic anhydride with the sulphoxide (13) to produce the acetoxylated derivative of formula (where Ac = acetyl): 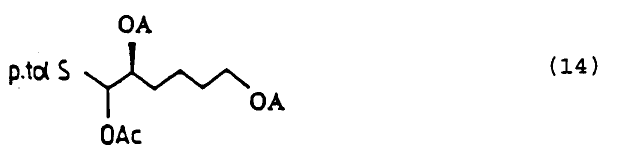
g) reaction with lithium tri(sec-butyl)borohydride to produce the alcohol of formula: 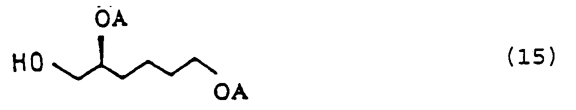
h) reaction with pyridinium chlorochromate to produce the aldehyde of formula: 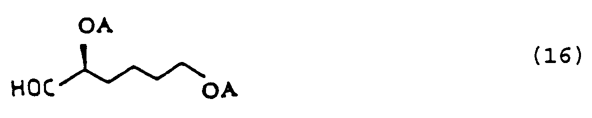
i) reaction with the compound of formula:
BrMg-≡-≡-H
to produce the diacetylene compound of formula (8).

11. Sulphoxide of formula: 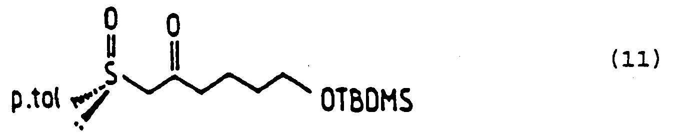 in which formula TBDMS represents the t-butyldimethylsilyl radical and tol the tolyl radical.

12. Sulphoxide of formula: 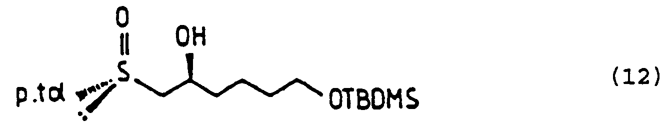 in which formula tol and TBDMS have the meanings given in Claim 11.

13. Sulphoxide of formula: 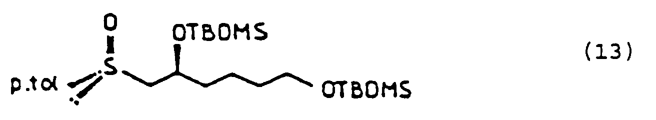 in which formula tol and TBDMS have the meanings given in Claim 11.

14. Acetoxylated derivative of formula: 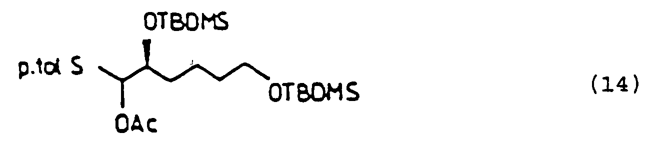 in which formula TBDMS and tol have the meanings given in Claim 11 and Ac represents the acetyl radical.

15. Alcohol of formula: 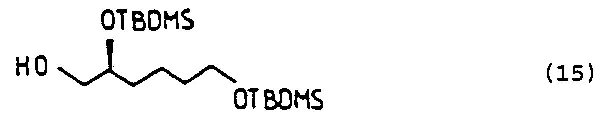 in which formula TBDMS represents the t-butyldimethylsilyl radical.

16. Aldehyde of formula: 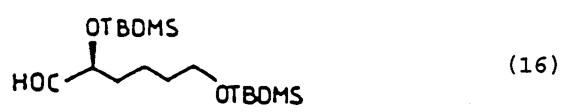 in which formula TBDMS represents the t-butyldimethylsilyl radical.

17. Diacetylene compound of formula: 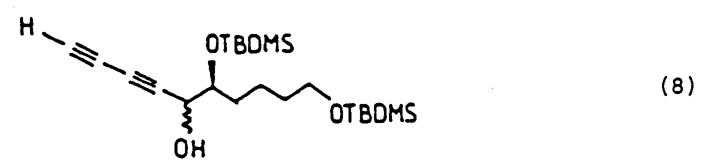 in which formula TBDMS represents the t-butyldimethylsilyl radical.

18. Diacetylene compound of formula: 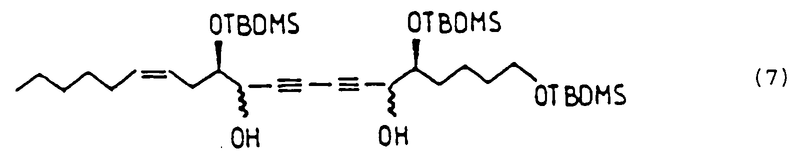 in which formula TBDMS represents the t-butyldimethylsilyl radical.

19. Butadienediol of formula: 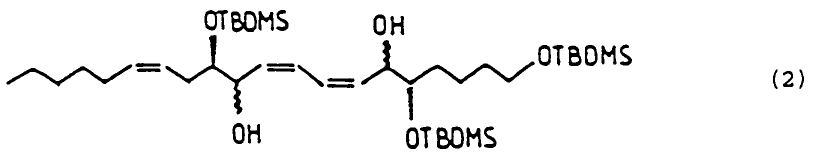 in which formula TBDMS represents the t-butyldimethylsilyl radical.

20. Dibenzoate of formula: 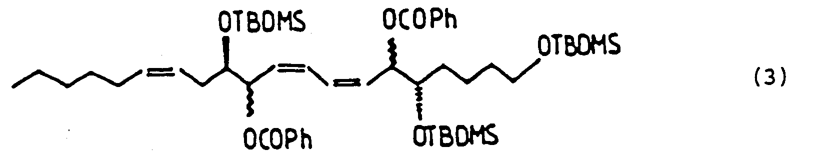 in which formula TBDMS represents the t-butyldimethylsilyl radical and Ph represents a phenyl radical.

21. Triether of formula: 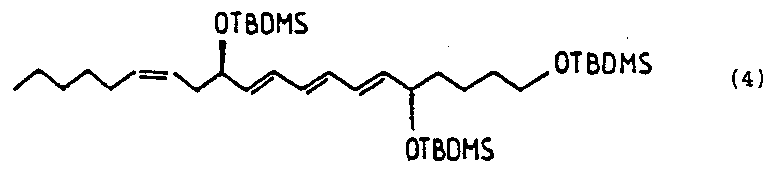 in which formula TBDMS represents the t-butyldimethylsilyl radical.

22. Triol of formula:

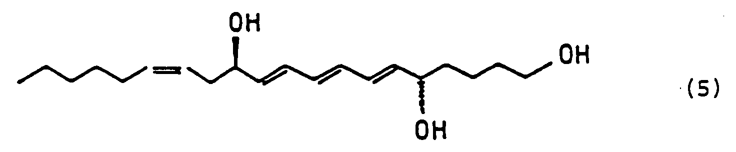

23. Lactone of formula:

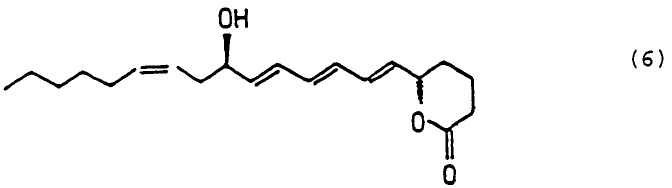

## Patentansprüche

1. Verfahren zur Herstellung von Leukotrien B₄ (LTB₄) in 6-trans-Form (6E) der Formel: 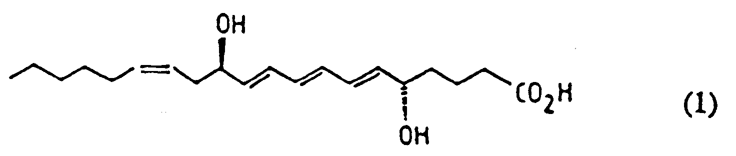 dadurch gekennzeichnet, daß man ein Butadiendiol der Formel: 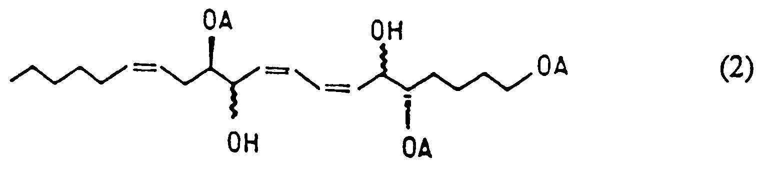 herstellt, worin der Rest A für eine silylierte Schutzgruppe steht; man die Hydroxylgruppen verestert, um eine Gruppe OCOB zu erhalten, worin der Rest B für einen Phenylrest oder einen C₁-C₆-Alkyl- oder -Alkoxy-substuierten Phenylrest steht, um den Diester der Formel: 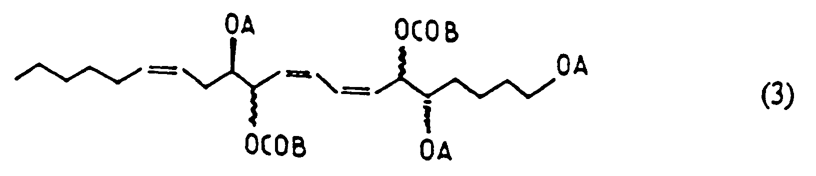 zu erhalten, und man diesen Diester einer reduktiven Eliminierung mit Hilfe von niedervalentem Titan [Ti(0)] oder einem Alkalimetallamalgam unterwirft, um den 6-trans-Triether der Formel: 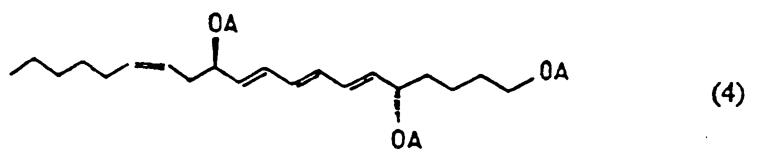 zu erhalten, an dem man dann die Gruppen -OA in den Positionen 5 und 12 in die Gruppen -OH und die Gruppe -CH₂OA in Position 1 in die Gruppe -COOH überführt, um das 6-trans-LTB₄ zu erhalten.

2. Verfahren nach Anspruch 1, dadurch gekennzeichnet, daß der Rest A eine t-Butyldimethylsilylgruppe ist.

3. Verfahren nach einem der Ansprüche 1 oder 2, dadurch gekennzeichnet, daß der Rest B ein Phenylrest ist.

4. Verfahren nach einem der Ansprüche 1 bis 3, dadurch gekennzeichnet, daß die reduktive Eliminierung mit Hilfe von niedervalentem Titan in einem Lösungsmittel bei einer Temperatur von 50 bis 70°C durchgeführt wird.

5. Verfahren nach Anspruch 4, dadurch gekennzeichnet, daß das Lösungsmittel Tetrahydrofuran ist.

6. Verfahren nach einem der Ansprüche 1 bis 5, dadurch gekennzeichnet, daß der Triether der Formel (4) durch folgende Schritte in (6E)-LTB₄ überführt wird:
a) Reaktion des 6-trans-Triethers der Formel (4) mit einer Tetra-n-butylammoniumfluorid-Lösung in einem Lösungsmittel bei einer Temperatur von -10 bis +20°C, um das Triol der Formel: 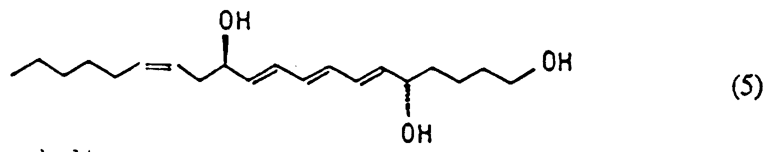 zu erhalten,
b) Reaktion des Triols der Formel (5) mit RuCl₂(PPh₃)₂ (worin Ph = Phenyl ist) unter Argon bei einer Temperatur von 10 bis 30°C, um das Lacton der Formel: 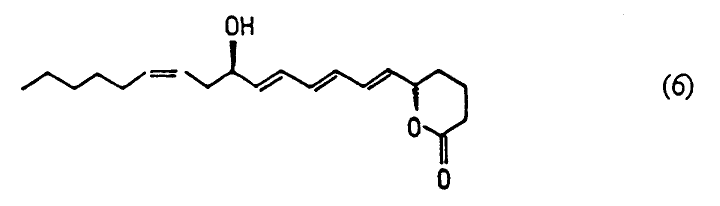 zu erhalten, und
c) Behandlung des Lactons der Formel (6) mit LiOH in gelöster Form in einem Lösungsmittel bei einer Temperatur unter 0°C.

7. Verfahren nach Anspruch 6, dadurch gekennzeichnet, daß das in den Schritten a) und/oder c) des Anspruchs 6 verwendete Lösungsmittel Tetrahydrofuran ist.

8. Verfahren nach einem der Ansprüche 1 bis 7, dadurch gekennzeichnet, daß das Butadiendiol der Formel (2) durch Hydrierung der diacetylenischen Verbindung der Formel: 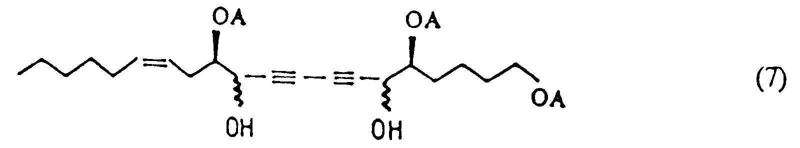 worin der Rest A die gleiche Bedeutung wie in Anspruch 1 besitzt, unter Argon in Gegenwart von aktiviertem Zn in Lösung in einem Wasser/Methanol-Gemisch hergestellt wird, wobei die diacetylenische Verbindung der Formel (7) ihrerseits hergestellt wird durch Reaktion der diacetylenischen Verbindung der Formel: 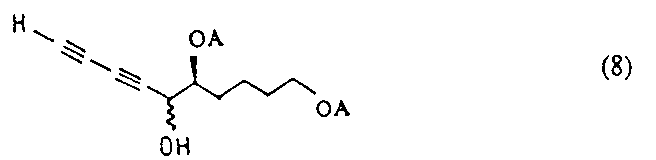 worin der Rest A die gleiche Bedeutung wie in Anspruch 1 besitzt, mit C₂H₅MgBr in einem Lösungsmittel unter Argon und dann mit dem Aldehyd der Formel (9): 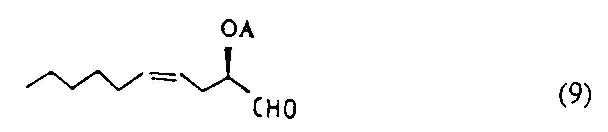 in gelöster Form in einem Lösungsmittel.

9. Verfahren nach Anspruch 8, dadurch gekennzeichnet, daß das Lösungsmittel Tetrahydrofuran ist.

10. Verfahren nach einem der Ansprüche 8 oder 9, dadurch gekennzeichnet, daß die diacetylenische Verbindung der Formel (8) durch folgende Schritte hergestellt wird:
a) Reaktion von Valerolacton mit Methanol in Gegenwart von Schwefelsäure, um Methyl-5-hydroxypentanoat zu erhalten;
b) Veretherung der Alkoholfunktion, um den Ether der Formel: 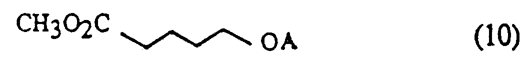 worin der Rest A die gleiche Bedeutung wie in Anspruch 1 besitzt, zu erhalten;
c) Zugabe von Methyl-p-tolylsulfoxid zum Ether der Formel (10) in Gegenwart von Lithiumdiisopropylamid in gelöster Form in einem Lösungsmittel bei einer Temperatur von -78 bis 20°C, um das Sulfoxid der Formel (11): 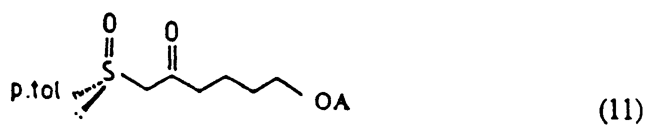 (worin tol = Tolyl ist) zu erhalten;
d) Reduktion der Carbonylgruppe der Verbindung der Formel (11) mit Diisobutylaluminiumhydrid bei einer Temperatur zwischen -70 und -85°C, um das Sulfoxid der Formel: 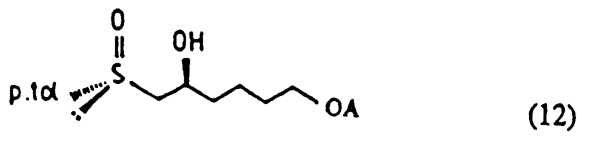 zu erhalten;
e) Veretherung der Alkoholfunktion des Sulfoxids der Formel (12) mit dem Chlorderivat der Formel ACl, worin der Rest A die gleiche Bedeutung wie in Anspruch 1 besitzt, in Gegenwart von Imidazol, um das Sulfoxid der Formel: 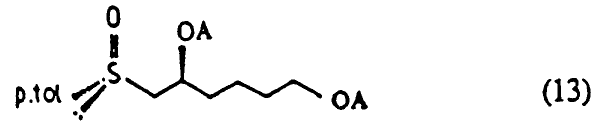 zu erhalten;
f) Reaktion von Natriumacetat und Essigsäureanhydrid mit dem Sulfoxid (13), um das acetoxylierte Derivat der Formel: 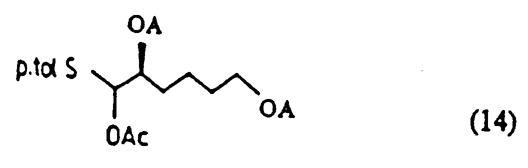 (worin Ac = Acetyl ist) zu erhalten;
g) Reaktion mit Lithium-tri-(sec-butyl)borhydrid, um den Alkohol der Formel: 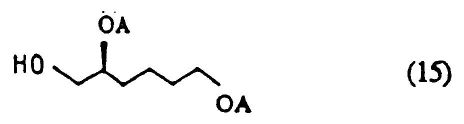 zu erhalten;
h) Reaktion mit Pyridiniumchlorchromat, um den Aldehyd der Formel: 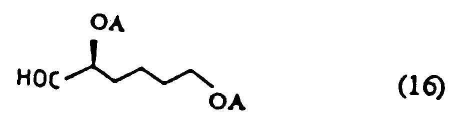 zu erhalten;
i) Reaktion mit der Verbindung der Formel:
BrMg-≡-≡-H
um die diacetylenische Verbindung der Formel (8) zu erhalten.

11. Sulfoxid der Formel: 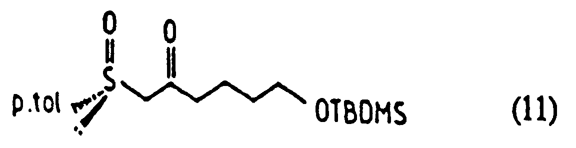 worin TBDMS für einen t-Butyldimethylsilylrest und tol für einen Tolylrest stehen.

12. Sulfoxid der Formel: 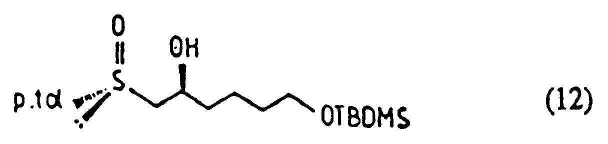 worin tol und TBDMS die in Anspruch 11 angegebenen Bedeutungen besitzen.

13. Sulfoxid der Formel: 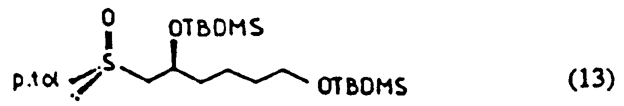 worin tol und TBDMS die in Anspruch 11 angegebenen Beeutungen besitzen.

14. Acetoxyliertes Derivat der Formel: 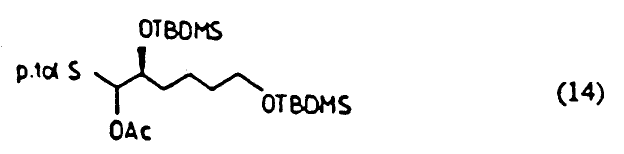 worin TBDMS und tol die in Anspruch 11 angegebenen Bedeutungen besitzen und Ac für einen Acetylrest stehen.

15. Alkohol der Formel: 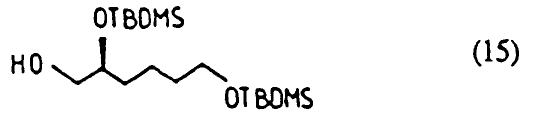 worin TBDMS für einen t-Butyldimethylsilylrest steht.

16. Aldehyd der Formel: 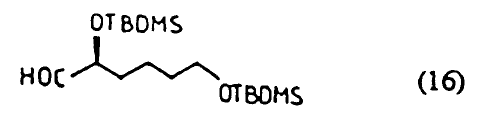 worin TBDMS für einen t-Butyldimethylsilylrest steht.

17. Diacetylenische Verbindung der Formel: 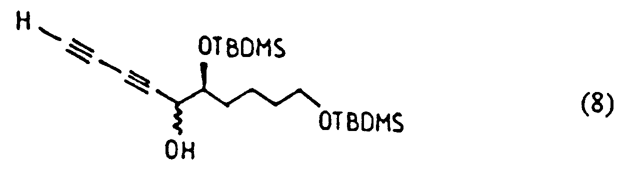 worin TBDMS für einen t-Butyldimethylsilylrest steht.

18. Diacetylenische Verbindung der Formel: 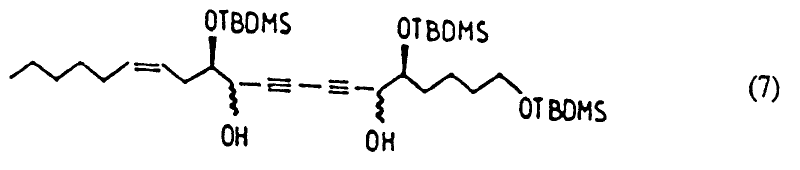 worin TBDMS für einen t-Butyldimethylsilylrest steht.

19. Butadiendiol der Formel: 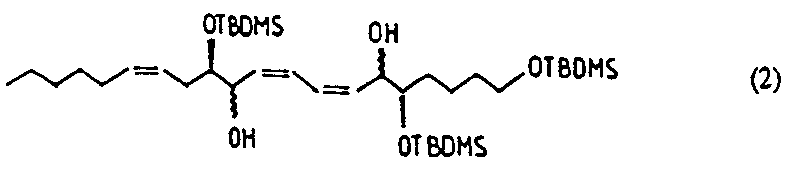 worin TBDMS für einen t-Butyldimethylsilylrest steht.

20. Dibenzoat der Formel: 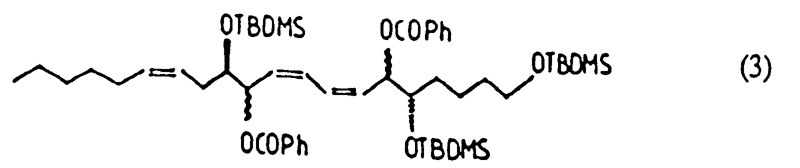 worin TBDMS für einen t-Butyldimethylsilylrest und Ph für einen Phenylrest steht.

21. Triether der Formel: 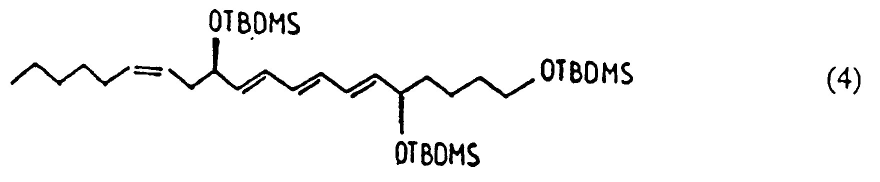 worin TBDMS für einen t-Butyldimethylsilylrest steht.

22. Triol der Formel:

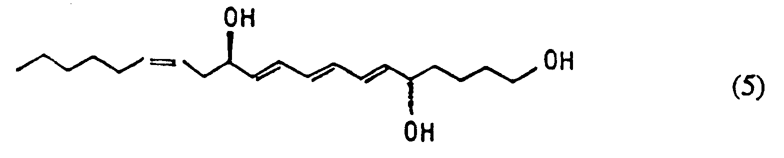

23. Lacton der Formel:
